(19) Europäisches Patentamt
European Patent Office
Office européen des brevets

(11) EP 2 103 614 A1

(12) EUROPEAN PATENT APPLICATION

(43) Date of publication:
**23.09.2009 Bulletin 2009/39**

(21) Application number: **08005046.1**

(22) Date of filing: **18.03.2008**

(51) Int Cl.:
***C07D 487/04*** (2006.01) ***A61P 1/00*** (2006.01)
***A61P 35/00*** (2006.01) ***A61P 21/00*** (2006.01)
***A61K 31/519*** (2006.01) ***A61K 31/4985*** (2006.01)
***A61K 31/5025*** (2006.01)

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Santhera Pharmaceuticals (Schweiz) AG**
**4410 Liestal (CH)**

(72) Inventors:
- **Soeberdt, Michael**
**79618 Rheinfelden (DE)**
- **Deppe, Holger**
**4052 Basel (CH)**
- **Abel, Ulrich**
**69126 Heidelberg (DE)**
- **Feurer, Achim**
**79424 Auggen (DE)**
- **Metz, Günther**
**79541 Lörrach (DE)**
- **Ott, Inge**
**68775 Ketsch (DE)**
- **Hoffmann-Enger, Barbara**
**4414 Füllinsdorf (CH)**
- **Nordhoff, Sonja**
**4144 Arlesheim (CH)**

(74) Representative: **Grünecker, Kinkeldey, Stockmair & Schwanhäusser Anwaltssozietät**
**Leopoldstrasse 4**
**80802 München (DE)**

(54) **Substituted imidazopyrimidine, imidazopyrazine and imidazopyridazine derivatives as melanocortin-4 receptor modulators**

(57) The present invention relates to substituted imidazopyrimidine derivatives, substituted imidazopyrazine derivatives and substituted imidazopyridazine derivatives as melanocortin-4 receptor (MC-4R) modulators, in particular as melanocortin 4 receptor antagonists. The antagonists are useful for the treatment of disorders and diseases such as cancer cachexia, muscle wasting, anorexia, emesis, anxiety, amytrophic lateral sclerosis and depression.

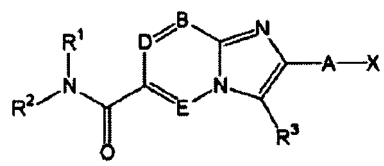

EP 2 103 614 A1

## Description

## Field of the Invention

The present invention relates to substituted imidazopyrimidine derivatives, substituted imidazopyrazine derivatives and substituted imidazopyridazine derivatives as melanocortin-4 receptor modulators. Depending on the structure and the stereochemistry, melanocortin-4 receptor modulators are either agonists or antagonists. The compounds of the invention are selective antagonists of the human melanocortin-4 receptor (MC-4R). The antagonists are useful for the treatment of disorders and diseases such as cancer cachexia, muscle wasting, anorexia, emesis, anxiety and depression.

## Background of the Invention

Melanocortins (MCs) stem from pro-opiomelanocortin (POMC) via proteolytic cleavage. These peptides, adrenocorticotropic hormone (ACTH), α-melanocyte-stimulating hormone (α-MSH), β-MSH and γ-MSH, range in size from 12 to 39 amino acids. The most important endogenous agonist for central MC-4R activation appears to be the tridecapeptide α-MSH. Among MCs, it was reported that α-MSH acts as a neurotransmitter or neuromodulator in the brain. MC peptides, particularly α-MSH, have a wide range of effects on biological functions including feeding behavior, pigmentation and exocrine function. The biological effects of α-MSH are mediated by a sub-family of 7-transmembrane G-protein-coupled receptors, termed melanocortin receptors (MC-Rs). Activation of any of these MC-Rs results in stimulation of cAMP formation.

To date, five distinct types of receptor subtype for MC (MC-1R to MC-5R) have been identified and these are expressed in different tissues.

MC-1R was first found in melanocytes. Naturally occurring inactive variants of MC-1 R in animals were shown to lead to alterations in pigmentation and a subsequent lighter coat color by controlling the conversion of phaeomelanin to eumelanin through the control of tyrosinase. From these and other studies, it is evident that MC-1R is an important regulator of melanin production and coat color in animals and skin color in humans.

The MC-2R is expressed in the adrenal gland representing the ACTH receptor. The MC-2R is not a receptor for α -MSH but is the receptor for the adrenocorticotropic hormone I (ACTH I).

The MC-3R is expressed in the brain (predominately located in the hypothalamus) and peripheral tissues like gut and placenta, and knock-out studies have revealed that the MC-3R may be responsible for alterations in feeding behavior, body weight and thermogenesis.

The MC-4R is primarily expressed in the brain. Overwhelming data support the role of MC-4R in energy homeostasis. Genetic knock-outs and pharmacologic manipulation of MC-4R in animals have shown that agonizing the MC-4R causes weight loss and antagonizing the MC-4R produces weight gain (A. Kask, et al., "Selective antagonist for the melanocortin-4 receptor (HS014) increases food intake in free-feeding rats," Biochem. Biophys. Res. Commun., 245: 90-93 (1998)).

MC-5R is ubiquitously expressed in many peripheral tissues including white fat, placenta and a low level of expression is also observed in the brain. However its expression is greatest in exocrine glands. Genetic knock-out of this receptor in mice results in altered regulation of exocrine gland function, leading to changes in water repulsion and thermoregulation. MC-5R knockout mice also reveal reduced sebaceous gland lipid production (Chen et al., Cell, 91: 789-798 (1997)).

Attention has been focused on the study of MC-3R and MC-4R modulators and their use in treating body weight disorders, such as obesity and anorexia. However, evidence has shown that the MC peptides have potent physiological effects besides their role in regulating pigmentation, feeding behavior and exocrine function. In particular, α-MSH recently has been shown to induce a potent anti-inflammatory effect in both acute and chronic models of inflammation including inflammatory bowel-disease, renal ischemia/reperfusion injury and endotoxin-induced hepatitis. Administration of α-MSH in these models results in substantial reduction of inflammation-mediated tissue damage, a significant decrease in leukocyte infiltration and a dramatic reduction in elevated levels of cytokines and other mediators to near baseline levels. Recent studies have demonstrated that the anti-inflammatory actions of α-MSH are mediated by MC-1 R. The mechanism by which agonism of MC-1R results in an anti-inflammatory response is likely through inhibition of the pro-inflammatory transcription activator, $NF\text{-}_KB$. $NF\text{-}_KB$ is a pivotal component of the pro-inflammatory cascade, and its activation is a central event in initiating many inflammatory diseases. Additionally, anti-inflammatory actions of α-MSH may be, in part, mediated by agonism of MC-3R and/or MC-5R.

A specific single MC-R that may be targeted for the control of obesity has not yet been identified, although evidence has been presented that MC-4R signaling is important in mediating feeding behavior (S.Q. Giraudo et al., "Feeding effects of hypothalamic injection of melanocortin-4 receptor ligands," Brain Research, 80: 302-306 (1998)). Further evidence for the involvement of MC-Rs in obesity includes: 1) the agouti ($A^{vy}$) mouse which ectopically expresses an antagonist of the MC-1R, MC-3R and MC-4R is obese, indicating that blocking the action of these three MC-R's can

lead to hyperphagia and metabolic disorders; 2) MC-4R knockout mice (D. Huszar et al., Cell, 88: 131-141 (1997)) recapitulate the phenotype of the agouti mouse and these mice are obese; 3) the cyclic heptapeptide melanotanin II (MT-II) (a non-selective MC-1R, -3R, -4R, and -5R agonist) injected intracerebroventricularly (ICV) in rodents, reduces food intake in several animal feeding models (NPY, ob/ob, agouti, fasted) while ICV injected SHU-9119 (MC-3R and 4R antagonist; MC-1R and -5R agonist) reverses this effect and can induce hyperphagia; 4) chronic intraperitoneal treatment of Zucker fatty rats with an α-NDP-MSH derivative (HP-228) has been reported to activate MC-1R, -3R, -4R, and -5R and to attenuate food intake and body weight gain over a 12 week period (I. Corcos et al., "HP-228 is a potent agonist of melanocortin receptor-4 and significantly attenuates obesity and diabetes in Zucker fatty rats," Society for Neuroscience Abstracts, 23: 673 (1997)).

MC-4R appears to play a role in other physiological functions as well, namely controlling grooming behavior, erection and blood pressure. Erectile dysfunction denotes the medical condition of inability to achieve penile erection sufficient for successful intercourse. The term "impotence" is often employed to describe this prevalent condition. Synthetic melanocortin receptor agonists have been found to initiate erections in men with psychogenic erectile dysfunction (H. Wessells et al., "Synthetic Melanotropic Peptide Initiates Erections in Men With Psychogenic Erectile Dysfunction: Double-Blind, Placebo Controlled Crossover Study," J. Urol., 160: 389-393, 1998). Activation of melanocortin receptors of the brain appears to cause normal stimulation of sexual arousal. Evidence for the involvement of MC-R in male and/or female sexual dysfunction is detailed in WO 00/74679.

Diabetes is a disease in which a mammal's ability to regulate glucose levels in the blood is impaired because the mammal has a reduced ability to convert glucose to glycogen for storage in muscle and liver cells. In Type I diabetes, this reduced ability to store glucose is caused by reduced insulin production. "Type II diabetes" or "Non-Insulin Dependent Diabetes Mellitus" (NIDDM) is the form of diabetes which is due to a profound resistance to insulin stimulating or regulatory effect on glucose and lipid metabolism in the main insulin-sensitive tissues, muscle, liver and adipose tissue. This resistance to insulin responsiveness results in insufficient insulin activation of glucose uptake, oxidation and storage in muscle, and inadequate insulin repression of lipolysis in adipose tissue and of glucose production and secretion in liver. When these cells become desensitized to insulin, the body tries to compensate by producing abnormally high levels of insulin and hyperinsulemia results. Hyperinsulemia is associated with hypertension and elevated body weight. Since insulin is involved in promoting the cellular uptake of glucose, amino acids and triglycerides from the blood by insulin sensitive cells, insulin insensitivity can result in elevated levels of triglycerides and LDL which are risk factors in cardiovascular diseases. The constellation of symptoms which includes hyperinsulemia combined with hypertension, elevated body weight, elevated triglycerides and elevated LDL, is known as Syndrome X. MC-4R agonists might be useful in the treatment of NIDDM and Syndrome X.

Among MC receptor subtypes, the MC4 receptor is also of interest in terms of the relationship to stress and the regulation of emotional behavior, as based on the following findings. Stress initiates a complex cascade of responses that include endocrine, biochemical and behavioral events. Many of these responses are initiated by release of corticotropin-releasing factor (CRF) (Owen MJ and Nemeroff CB (1991) Physiology and pharmacology of corticotrophin releasing factor. Pharmacol Rev 43: 425-473). In addition to activation of the brain CRF system, there are several lines of evidence that melanocortins (MCs), which stem from proopiomelanocortin by enzymatic processing, mediate important behavioral and biochemical responses to stress and, consequently, stress-induced disorders like anxiety and depression (Anxiolytic-Like and Antidepressant-Like Activities of MCL0129 (1-[(S)-2-(4-Fluorophenyl)-2-(4-isopropylpiperadin-1-yl) ethyl]-4-[4-(2-methoxynaphthalen-1-yl)butyl]piperazine), a Novel and Potent Nonpeptide Antagonist of the Melanocortin-4 Receptor; Shigeyuki Chaki et al, J. Pharm. Exp. Ther. (2003) 304(2), 818-26).

Chronic diseases, such as malignant tumors or infections, are frequently associated with cachexia resulting from a combination of a decrease in appetite and a loss of lean body mass. Extensive loss of lean body mass is often triggered by an inflammatory process and is usually associated with increased plasma levels of cytokines (e.g. TNF-α), which increase the production of α-MSH in the brain. Activation of MC4 receptors in the hypothalamus by α-MSH reduces appetite and increases energy expenditure. Experimental evidence in tumor bearing mice suggests that cachexia can be prevented or reversed by genetic MC4 receptor knockout or MC4 receptor blockade. The increased body weight in the treated mice is attributable to a larger amount of lean body mass, which mainly consists of skeletal muscle (Marks D.L. et al. Role of the central melanocortin system in cachexia. Cancer Res. (2001) 61: 1432-1438).

Internal studies in ferrets have shown that a pharmacological inhibition of melanocortin-4 receptors strongly inhibits emesis. Accordingly, MC-4R inhibitors could be used to treat emesis, especially in patients undergoing chemotherapy.

Clinical observations indicate, that progression of Amytrophic Lateral Sclerosis (ALS) might be inversely correlated with body weight (e.g. Ludolph AC, Neuromuscul. Disord. (2006) 16 (8):530-8). Accordingly, MC-4R inhibitors could be used to treat ALS patients.

Melanocortin-4-receptor modulators have been previously described in the literature. For example, substituted phenylpiperidine derivatives have been synthesized and explored as MC-4R agonists as well as antagonists.

In view of the unresolved deficiencies in treatment of various diseases and disorders as discussed above, it is

an object of the present invention to provide novel compounds with improved ability to cross the blood brain barrier, which are useful as melanocortin-4 receptor antagonists to treat cancer cachexia, muscle wasting, emesis, amytrophic lateral sclerosis, anorexia, anxiety, depression and other diseases with MC-4R involvement.

Surprisingly, it has been found that novel imidazopyrimidines, imidazopyrazines and imidazopyridazines according to formula (I) shown below solve the object of the present invention.

## Summary of the Invention

The present invention relates to substituted imidazopyrimidine derivatives, substituted imidazopyrazine derivatives and substituted imidazopyridazine derivatives of structural

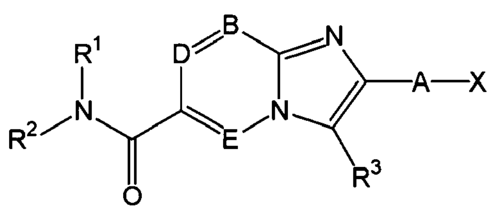

(I)

formula (I)
wherein $R^1$, $R^2$, $R^3$, A, B, D, E and X are defined as described below.

The imidazopyrimidine, imidazopyrazine and imidazopyridazine derivatives of structural formula (I) are effective as melanocortin receptor modulators and are particularly effective as selective melanocortin-4 receptor (MC-4R) antagonists. They are therefore useful for the treatment of disorders where the inactivation of the MC-4R is involved. The antagonists are useful for the treatment of disorders and diseases such as cancer cachexia, muscle wasting, anorexia, emesis, amytrophic lateral sclerosis, anxiety and depression.

The present invention also relates to pharmaceutical compositions comprising the compounds of the present invention and a pharmaceutically acceptable carrier.

## Detailed Description of the Invention

The present invention relates to substituted imidazopyrimidine derivatives, substituted imidazopyrazine derivatives and substituted imidazopyridazine derivatives useful as melanocortin receptor modulators, in particular, as selective MC-4R antagonists.

Substituted N-benzyl-N-methyl-2-phenyl-5-diethylamido-3-methylamino-imidazo[1,2-a] pyridines are known from WO-A-02/066478 which describes antagonists of gonadotropin releasing hormone. The present invention relates to novel imidazopyrimidines, imidazopyrazines and imidazopyridazines which are used as antagonists of MC-4R.

The compounds of the present invention are represented by structural formula (I)

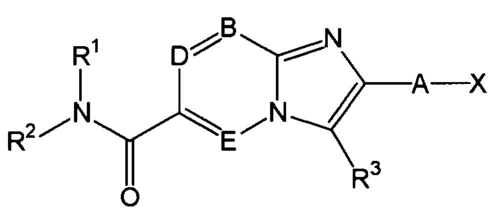

(I)

and enantiomers, diastereomers, tautomers, solvates and pharmaceutically acceptable salts thereof, wherein

$R^1$ and $R^2$ are independently from each other selected from

H,
$C_{1-6}$ alkyl,
$C_{1-6}$ alkylene-O-$C_{1-6}$alkyl
$C_{1-3}$ alkylene-heterocyclyl, and
$C_{1-6}$ alkylene-$C_{3-7}$cycloalkyl, or

$R^1$ and $R^2$, together with the nitrogen atom to which they are attached to, form a 5 to 6-membered ring which may additionally contain one oxygen atom in the ring and which is unsubstituted or substituted by one or more substituents selected from OH, $C_{1-6}$alkyl, O-$C_1$-$_6$alkyl, $C_{0-3}$alkylene-$C_{3-5}$cycloalkyl, $C_{1-6}$alkylene-O-$C_1$-$_6$alkyl and $(CH_2)_{0-3}$-phenyl;
B, D and E are independently from each other selected form CH and N with the proviso that one of B, D and E represents N;
A is -NH-,

-$C_{1-6}$alkylene,
-$C_{2-6}$alkenylene,
-$C_{2-6}$alkinylene or
a bond

wherein alkylene, alkenylene and alkinylene are unsubstituted or substituted with one ore more $R^7$;
$R^7$ is independently selected from

$C_1$-$_6$alkyl,
$OR^{14}$,
$NR^{15a}R^{15b}$,
halogen,
phenyl and
heteroaryl,

wherein phenyl and heteroaryl are unsubstituted or substituted by 1 to 3 $R^{4a}$;
X is H,

CN,
$C_3$-$_8$cydoalkyl, unsubstituted or substituted with one or more halogen atoms,
phenyl,
phenyl which is fused with a saturated heterocyclic 6-membered ring, wherein the heterocyclic ring contains 1 or 2 heteroatoms independently selected from O and N,
4 to 8-membered saturated or unsaturated heterocyclyl containing 1 to 4 heteroatoms independently selected from N, O and S,
5- to 6-membered heteroaryl containing 1 to 4 heteroatoms independently selected from N, O and S,
-C(O)-$R^6$,
-$OR^{14}$,
halogen or
$NR^{15a}R^{15b}$,

wherein each phenyl, heterocyclyl and heteroaryl is unsubstituted or substituted by 1 to 3 $R^{4a}$ and/or 1 $R^{4b}$ and/or 1 $R^5$;
$R^{4a}$ ishalogen,

CN,
$C_{1-6}$alkyl, unsubstituted or substituted with one or more substituents independently selected from halogen, OH and O-$C_{1-6}$alkyl,
O-$C_{1-6}$alkyl, wherein alkyl is unsubstituted or substituted with one or more halogen atoms, or
OH;

$R^{4b}$ is $C(O)NH_2$,

C(O)NH-$C_{1-6}$alkyl,
C(O)N-$(C_{1-6}alkyl)_2$,
$SO_2$-$C_{1-6}$alkyl,
C(O)NH-$SO_2$-$C_{1-6}$alkyl,

oxo, whereby the ring is at least partially saturated,
$NH_2$,
NH-$C_{1-6}$alkyl,
N-($C_{1-6}$alkyl)$_2$,
NH-$SO_2$-$CH_3$, or
NH-$SO_2$-$CF_3$;

$R^5$ is 5 to 6-membered saturated or unsaturated heterocyclyl containing 1 to 3

heteroatoms independently selected from N, O and S or
5 to 6-membered heteroaryl containing 1 to 3 heteroatoms independently selected from N, O and S

wherein each heterocyclyl and heteroaryl is unsubstituted or substituted by 1 or 2

$R^{4a}$;
$R^6$ is H,
OH,
$C_{1-6}$alkyl, unsubstituted or substituted with one or more halogen atoms,
O-$C_{1-6}$alkyl, wherein alkyl is unsubstituted or substituted with one or more $R^{16}$,
phenyl,
4 to 8-membered saturated or unsaturated heterocyclyl containing 1 to 3
heteroatoms selected from N, O and S,
or
$NR^{16a}R^{16b}$,

wherein each phenyl and heterocyclyl is unsubstituted or substituted by 1 to 3 $R^{4a}$
and/or $R^5$;
$R^3$ is -$(CR^8R^9)_n$-T;
$R^8$ and $R^9$ are independently from each other selected from

H,
OH,
halogen,
$C_{1-6}$alkyl, and
O-$C_{1-6}$alkyl,
n is 1 to 6;
T is

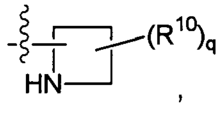 , 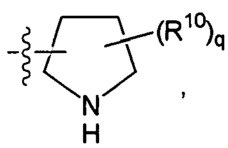 , 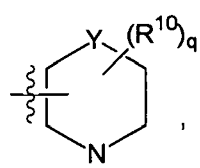 ,

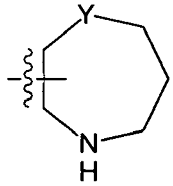 , 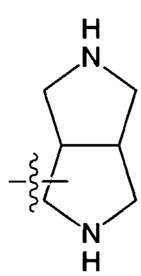 , 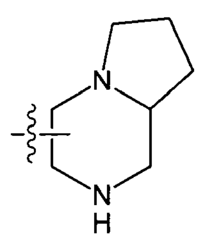

or $NR^{12}R^{13}$;

$R^{10}$ is H,

$NH_2$,
OH,
$OC_{1-6}$alkyl,

wherein alkyl is unsubstituted or substituted by 1 to 3 halogens

$C_{1-6}$alkyl,
halogen,
$NH(C_{1-6}alkyl)$,
$N(C_{1-6}alkyl)_2$,
phenyl or
heteroaryl,

wherein phenyl and heteroaryl are unsubstituted or substituted by 1 to 3 $R^{4a}$;
q is 1 or 2;
Y is $CH_2$,

$NR^{11}$ or
O;

$R^{11}$ is H,

$C_1$-$_6$alkyl or
$(CH_2)_{0-6}$-$C_{3-7}$cyctoalkyl;

$R^{12}$ and $R^{13}$ are independently from each other selected from

H,
$C_{1-6}$ alkyl,
$(CH_2)_{0-2}$-$C_{3-7}$cycloalkyl and
$C_{1-6}$alkylene-O-$C_{1-6}$alkyl;

wherein alkyl, alkylene and cycloalkyl are unsubstituted or substituted by 1 to 3 $R^{4a}$;
$R^{14}$ is H,

$C_1$-$_6$alkyl, unsubstituted or substituted with one or more substituents selected from
halogen,
phenyl or
heteroaryl,

wherein phenyl and heteroaryl are unsubstituted or substituted by 1 to 3 $R^{4a}$;
$R^{15a}$ and $R^{15b}$ are independently from each other selected from

H,
$C_{1-6}$alkyl, unsubstituted or substituted with one or more substituents selected from
halogen, OH, $O(C_{1-6}alkyl)$, $NH_2$, $NH(C_{1-6}alkyl)$ and $N(C_{1-6}alkyl)_2$,
phenyl and
heteroaryl,

wherein phenyl and heteroaryl are unsubstituted or substituted by 1 to 3 $R^{4a}$, and $C(O)C_{1-6}$alkyl;
$R^{16}$ $R^{16a}$ and $R^{16b}$ are independently from each other selected from

H,
$C_{1-6}$alkyl, unsubstituted or substituted with one or more substituents selected from
halogen, OH, $O(C_{1-6}alkyl)$, $NH_2$, $NH(C_{1-6}alkyl)$ and $N(C_{1-6}alkyl)_2$,
$C_{0-3}$alkylene-$C_{3-5}$cycloalkyl,

phenyl and
heteroaryl,

wherein phenyl and heteroaryl are unsubstituted or substituted by 1 to 3 $R^{4a}$.

In a preferred embodiment, the variant A represents -NH- or a bond. More preferably, A represents a bond.

In an equally preferred embodiment, the variant A represents $-C_{1-6}$alkylene, $-C_{2-6}$ alkenylene or $-C_{2-6}$alkinylene wherein alkylene, alkenylene and alkinylene are unsubstituted or substituted with one ore more $R^7$ such as 1, 2 or 3 $R^7$. Preferably, A represents $C_{1-3}$alkylene, such as methylene, ethylene, propylene or isopropylene, $C_{2-3}$alkenylene, such as ethenylene or prop-1-enylene, or $C_{2-3}$alkinylene, such as ethinylene or prop-2-inylene. It is further preferred that alkylene, alkenylene and alkinylene are unsubstituted or substituted by 1 $R^7$. More preferably, alkylene, alkenylene and alkinylene are unsubstituted.

$R^7$ is as defined above. Preferably, $R^7$ represents $C_{1-6}$alkyl, $OR^{14}$, $NR^{15a}R^{15b}$ or halogen, wherein $R^{14}$, $R^{15a}$ and $R^{15b}$ are defined as above. More preferably, $R^7$ represents $C_{1-6}$alkyl, OH, $NH_2$ or fluorine.

It is further preferred that $R^1$ and $R^2$ independently from each other represent $C_{3-6}$alkyl or that $R^1$ and $R^2$, together with the nitrogen atom to which they are attached to, form a 5 to 6-membered ring which may additionally contain one oxygen atom in the ring and which is unsubstituted or substituted by one or more substituents, preferably 1, 2 or 3 substituents, independently selected from OH, $C_{1-6}$alkyl, $C_{0-3}$alkylene-$C_{3-5}$cycloalkyl, O-$C_{1-6}$alkyl, $C_{1-6}$alkylene-O-$C_{1-6}$alkyl and $(CH_2)_{0-3}$-phenyl.
More preferably, $R^1$ and $R^2$ independently from each other represent $C_{3-6}$alkyl.

Preferably, B represents a nitrogen atom whereas D and E mean a CH group or, alternatively, D represents a nitrogen atom whereas B and E mean a CH group.

In a preferred embodiment, the variant T is $NR^{12}R^{13}$. Therein, the variants $R^{12}$ and $R^{13}$ are preferably independently from each other selected from H, $C_{1-3}$alkyl and $(CH_2)_{0-2}$-$C_{3-6}$ cycloalkyl, wherein alkyl and cycloalkyl are unsubstituted or substituted by 1 to 3 $R^{4a}$ such as 1, 2 or 3 substituents $R^{4a}$ and wherein $R^{4a}$ is defined as above

In an alternative preferred embodiment, the variant T is selected from

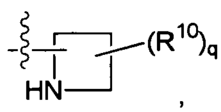 , 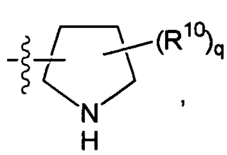 , 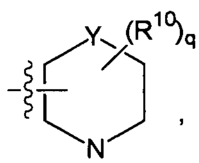 ,

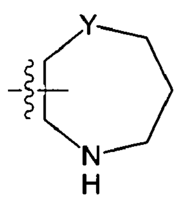 ,

It is preferred that the variant Y is $CH_2$ or $NR^{11}$ wherein $R^{11}$ is defined as above. Preferably, $R^{11}$ is hydrogen.

It is further preferred that $R^{10}$ is selected from H, $NH_2$, $C_{1-6}$alkyl, $NH(C_{1-6}alkyl)$ and $N(C_{1-6}\ alkyl)_2$. More preferably, $R^{10}$ is H, $NH_2$ or $C_{1-6}$alkyl.

Regarding the variant X, said variant preferably represents a 4 to 8-membered saturated or unsaturated heterocyclyl containing 1, 2, 3 or 4 heteroatoms independently selected from N, O and S, or a 5- to 6-membered heteroaryl containing 1, 2, 3 or 4 heteroatoms independently selected from N, O and S, wherein each heterocyclyl or heteroaryl is unsubstituted or substituted by 1 to 3 $R^{4a}$ such as 1, 2 or 3 $R^{4a}$ and/or 1 $R^{4b}$ and/or 1 $R^5$ wherein $R^{4a}$, $R^{4b}$ and $R^5$ are defined as above.

More preferably, X represents

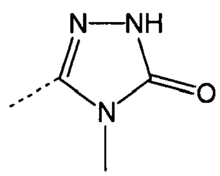 , 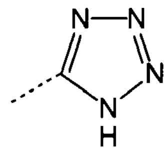 , 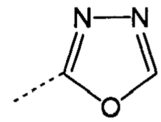 ,

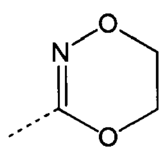 , 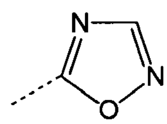 , 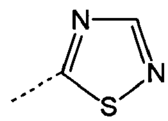 ,

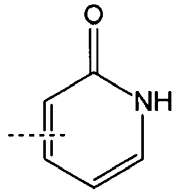 , 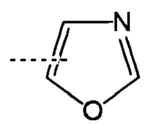 or 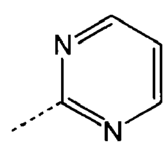 ,

wherein each heterocyclyl or heteroaryl is unsubsubstituted or substituted by 1 to 3 $R^{4a}$ such as 1, 2 or 3 $R^{4a}$ and/or 1 $R^{4b}$ and/or 1 $R^5$ wherein $R^{4a}$, $R^{4b}$ and $R^5$ are defined as above. Preferably, the rings shown in the above preferred embodiment of X are substituted by 1 or 2 $R^{4a}$, preferably 1 $R^{4a}$. More preferably, $R^{4a}$ is selected from $C_{1-6}$ alkyl, unsubstituted or substituted with one or more substituents selected from halogen, OH and O-$C_{1-6}$alkyl.

In an alternative embodiment, the variant X represents phenyl which is unsubstituted or substituted by 1 to 3 $R^{4a}$ such as 1, 2 or 3 $R^{4a}$ and/or 1 $R^{4b}$ and/or 1 $R^5$ wherein $R^{4a}$, $R^{4b}$ and $R^5$ are defined as above.

In an equally preferred embodiment, the variant X represents -C(O)-$R^6$, $OR^{14}$, or $NR^{15a}R^{15b}$ wherein $R^6$, $R^{14}$, $R^{15a}$ and $R^{15b}$ are defined as above.. More preferably, X is -C(O)-$R^6$ or $NR^{15a}R^{15b}$, most preferably X is -C(O)-$R^6$.

$R^6$ is preferably O-$C_{1-6}$alkyl, wherein alkyl is unsubstituted or substituted with one or more $R^{16}$.

In an equally preferred embodiment, the variant $R^6$ is $NR^{16a}R^{16b}$, more preferably NH-$C_{1-6}$ alkyl.

The index n represents an integer from 1 to 6, such as the integers 1, 2, 3, 4, 5 or 6. Preferably, n represents the integers 1, 2, 3, or 4.

The index q represents the integers 1 or 2. Preferably, q is 1.

Compounds of the formula (I) in which some or all of the above-mentioned groups have the preferred or more preferred meanings are also an object of the present invention.

In the above and the following, the employed terms have the meaning as described below:

Alkyl is a straight chain or branched alkyl having 1, 2, 3, 4, 5 or 6 carbon atoms, such as methyl, ethyl, n-propyl, isopropyl, n-butyl, isobutyl, tert-butyl, n-pentyl, isopentyl, neopentyl, or hexyl.

Alkenyl is a straight chain or branched alkyl having 1, 2, 3, 4, 5, or 6 carbon atoms and one to three double bonds, preferably one or two double bonds, most preferably one double bound. Preferred examples of a $C_{2-6}$alkenyl group are ethenyl, prop-1-enyl, prop-2-enyl, isoprop-1-enyl, n-but-1-enyl, n-but-2-enyl, n-but-3-enyl, isobut-1-enyl, isobut-2-enyl, n-pent-1-enyl, n-pent-2-enyl, n-pent-3-enyl, n-pent-4-enyl, n-pent-1,3-enyl, isopent-1-enyl, isopent-2-enyl, neopent-1-enyl, n-hex-1-enyl, n-hex-2-enyl, n-hex-3-enyl, n-hex-4-enyl, n-hex-5-enyl, n-hex-1,3-enyl, n-hex-2,4-enyl, n-hex-3,5-enyl, and n-hex-1,3,5-enyl. More preferred examples of a $C_{2-6}$alkenyl group are ethenyl and prop-1-enyl.

Alkinyl is a straight chain or branched alkyl having 1, 2, 3, 4, 5, or 6 carbon atoms and one to three triple bonds, preferably one or two triple bonds, most preferably one triple bond. Preferred examples of a $C_{2-6}$alkinyl group are ethinyl, prop-1-inyl, prop-2-inyl, n-but-1-inyl, n-but-2-inyl, n-but-3-inyl, n-pent-1-inyl, n-pent-2-inyl, n-pent-3-inyl, n-pent-4-inyl, n-pent-1,3-inyl, isopent-1-inyl, neopent-1-inyl, n-hex-1-inyl, n-hex-2-inyl, n-hex-3-inyl, n-hex-4-inyl, n-hex-5-inyl, n-hex-1,3-inyl, n-hex-2,4-inyl, n-hex-3,5-inyl and n-hex-1,3,5-inyl. More preferred examples of a $C_{2-6}$alkinyl group are ethinyl and prop-1-inyl.

Cycloalkyl is an alkyl ring having preferably 3, 4, 5, 6, 7 or 8 carbon atoms at the most, such as cyclopropyl,

cyclobutyl, cyclopentyl, cyclohexyl, cycloheptyl or cyclooctyl, more preferably 3, 4, 5 or 6 carbon atoms.

Heteroaryl is an aromatic moiety having 1, 2, 3, 4 or 5 carbon atoms and at least one heteroatom independently selected from O, N and/or S. Heteroaryl is preferably selected from thienyl, pyrrolyl, imidazolyl, pyrazolyl, pyridyl, pyrazinyl, pyrimidinyl, pyridazinyl, isothiazolyl, isoxazyl, furanyl, and indazolyl, more preferably from thienyl, furanyl, imidazolyl, pyridyl, and pyrimidinyl.

Heterocyclyl is a saturated or unsaturated ring containing at least one heteroatom independently selected from O, N and/or S and 1, 2, 3, 4, 5, 6 or 7 carbon atoms. Preferably, heterocyclyl is a 4, 5, 6, 7 or 8-membered ring and is preferably selected from tetrahydrofuranyl, azetidinyl, pyrrolidinyl, piperidinyl, pyranyl, morpholinyl, thiomorpholinyl, more preferably from piperidinyl and pyrrolidinyl.

Halogen is a halogen atom selected from F, Cl, Br and I, preferably from F, Cl and Br.

The compounds of structural formula (I) are effective as melanocortin receptor modulators and are particularly effective as selective modulators of MC-4R. They are useful for the treatment and/or prevention of disorders responsive to the inactivation of MC-4R, such as cancer cachexia, muscle wasting, anorexia, emesis, amytrophic lateral sclerosis, anxiety, depression and other diseases with MC-4R involvement.

## Optical Isomers - Diastereomers - Geometric Isomers - Tautomers

Compounds of structural formula (I) contain one or more asymmetric centers and can occur as racemates and racemic mixtures, single enantiomers, diastereomeric mixtures and individual diastereomers. The present invention is meant to comprehend all such isomeric forms of the compounds of structural formula (I).

Compounds of structural formula (I) may be separated into their individual diastereoisomers by, for example, fractional crystallization from a suitable solvent, for example methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration.

Alternatively, any stereoisomer of a compound of the general formula (I) may be obtained by stereospecific synthesis using optically pure starting materials or reagents of known absolute configuration.

## Salts

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, furnaric, gluconic, glutamic, hydrobromic, hydrochloric, isethionic, lactic, maleic, malic, mandelic, methanesulfonic, malonic, mucic, nitric, parnoic, pantothenic, phosphoric, propionic, succinic, sulfuric, tartaric, p-toluenesulfonic, trifluoroacetic acid and the like.

Particularly preferred are citric, fumaric, hydrobromic, hydrochloric, maleic, phosphoric, sulfuric and tartaric acids.

It will be understood that, as used herein, references to the compounds of formula (I) are meant to also include the pharmaceutically acceptable salts.

## Utility

Compounds of formula (I) are melanocortin receptor antagonists and as such are useful in the treatment, control or prevention of diseases, disorders or conditions responsive to the inactivation of one or more of the melanocortin receptors including, but not limited to, MC-1R, MC-2R, MC-3R, MC-4R or MC-5R. Such diseases, disorders or conditions include, but are not limited to, cancer cachexia, muscle wasting, anorexia, emesis, amytrophic lateral sclerosis, anxiety and depression.

The compounds of formula (I) can be further used in the treatment, control or prevention of hypertension, hyperlipidemia, osteoarthritis, cancer, gall bladder disease, sleep apnea, compulsion, neuroses, insomnia/sleep disorder, substance abuse, pain, fever, inflammation, immune-modulation, rheumatoid arthritis, skin tanning, acne and other skin disorders, neuroprotective and cognitive and memory enhancement including the treatment of Alzheimer's disease.

## Administration and Dose Ranges

Any suitable route of administration may be employed for providing a mammal, especially a human with an effective dosage of a compound of the present invention. For example, oral, rectal, topical, parenteral, ocular, pulmonary, nasal and the like may be employed. Dosage forms include tablets, troches, dispersions, suspensions, solutions, capsules, creams, ointments, aerosols and the like. Preferably compounds of formula (I) are administered orally or topically.

The effective dosage of active ingredient employed may vary depending on the particular compound employed, the mode of administration, the condition being treated and the severity of the condition being treated. Such dosage may be ascertained readily by a person skilled in the art.

When treating cancer cachexia, muscle wasting, anorexia, emesis, amytrophic lateral sclerosis, anxiety and depression generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

## Formulation

The compounds of formula (I) are preferably formulated into a dosage form prior to administration. Accordingly the present invention also includes a pharmaceutical composition comprising a compound of formula (I) and a suitable pharmaceutical carrier.

The present pharmaceutical compositions are prepared by known procedures using well-known and readily available ingredients. In making the formulations of the present invention, the active ingredient (a compound of formula (I)) is usually mixed with a carrier, or diluted by a carrier, or enclosed within a carrier, which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient.

## Preparation of Compounds of the Invention

The compounds of formula (I) when existing as a diastereomeric mixture, may be separated into diastereomeric pairs of enantiomers by fractional crystallization from a suitable solvent such as methanol, ethyl acetate or a mixture thereof. The pair of enantiomers thus obtained may be separated into individual stereoisomers by conventional means by using an optically active acid as a resolving agent. Alternatively, any enantiomer of a compound of the formula (I) may be obtained by stereospecific synthesis using optically pure starting materials or reagents of known configuration.

The compounds of formula (I) of the present invention can be prepared according to the procedures of the following Schemes and Examples, using appropriate materials and are further exemplified by the following specific examples. Moreover, by utilizing the procedures described herein, in conjunction with ordinary skills in the art, additional compounds of the present invention claimed herein can be readily prepared. The compounds illustrated in the examples are not, however, to be construed as forming the only genus that is considered as the invention. The Examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described previously. The free amine bases corresponding to the isolated salts can be generated by neutralization with a suitable base, such as aqueous sodium hydrogencarbonate, sodium carbonate,

sodium hydroxide and potassium hydroxide, and extraction of the liberated amine free base into an organic solvent followed by evaporation. The amine free base isolated in this manner can be further converted into another pharmaceutically acceptable salt by dissolution in an organic solvent followed by addition of the appropriate acid and subsequent evaporation, precipitation or crystallization. All temperatures are degrees Celsius.

In the schemes, preparations and examples below, various reagent symbols and abbreviations have the following meanings

| | |
|---|---|
| AcOH | acetic acid |
| $Ac_2O$ | acetic anhydride |
| CDI | 1,1'-carbonyldiimidazole |
| dba | dibenzylidenacetone |
| DCM | dichloromethane |
| DCE | 1,2-dichloroethane |
| DIEA | ethyl-diisopropylamine |
| DMF | N,N-dimethylformamide |
| DMSO | dimethylsulfoxide |
| EDC | 1-(3-dimethylaminopropyl)-3-ethylcarbodiimide hydrochloride |
| $Et_2O$ | diethyl ether |
| EtOAc | ethyl |
| HOAc | acetic acid |
| HOBt | 1-hydroxybenzotriazole |
| h | hour(s) |
| MeCN | acetonitrile |
| MeOH | methanol |
| Ms | mesyl |
| MW | molecular weight |
| NMM | N-methylmorpholine |
| Pg | protecting group |
| TEA | triethylamine |
| TFA | trifluoroacetic acid |
| TFAA | trifluoroacetic acid anhydride |
| THF | tetrahydrofurane |
| TMSI | trimethylsilyliodide |

| | |
|---|---|
| $t_R$ (min) | HPLC retention time |
| Ts | tosyl |
| Z | benzyloxycarbonyl |

**Reaction Scheme 1:**

**Synthesis of 2-sulfonylamino-pyrimidine-5-carboxylic acid amides and 2-sulfonyl-amino-pyrazine-5-carboxylic acid amides**

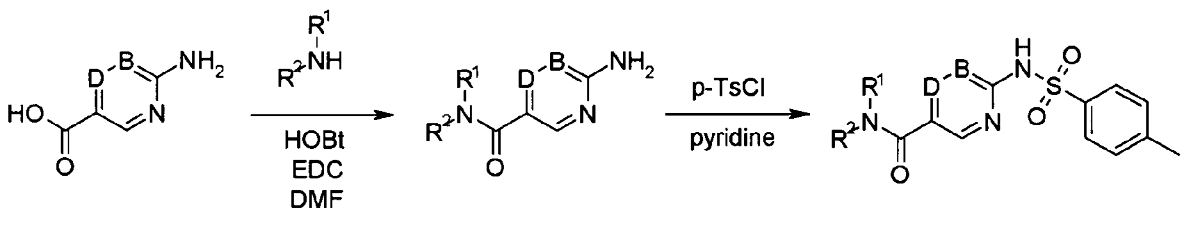


As shown in Reaction Scheme 1, optionally substituted amine and 2-amino-pyrimidine-5-carboxylic acid or 5-amino-pyrazine-2-carboxylic acid are reacted in an amide coupling reaction in the presence of a coupling reagent such as EDC in an organic solvent such as DMF or DCM at a suitable temperature. The resulting amide can then be reacted

with a sulfonylchloride in a solvent such as pyridine or any other appropriate solvent and an organic base such as triethylamine to yield the corresponding sulfonylamino-amides.

### Reaction Scheme 2:

### Synthesis of 2-sulfonylamino-pyrimidine-5-carboxylic acid methyl ester and 2-sulfonyl-amino-pyrazine-5-carboxylic acid methyl ester

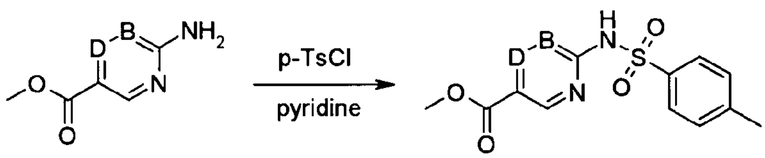


Alternatively, 2-amino-pyrimidine-5-carboxylic acid methyl ester or 5-amino-pyrazine-2-carboxylic acid methyl ester can be reacted under the conditions described above to yield the corresponding sulfonylamino-esters, as shown in Reaction Scheme 2.

### Reaction Scheme 3:

### Synthesis of 6-sulfonylamino-pyridazine-3-carboxylic acid amide

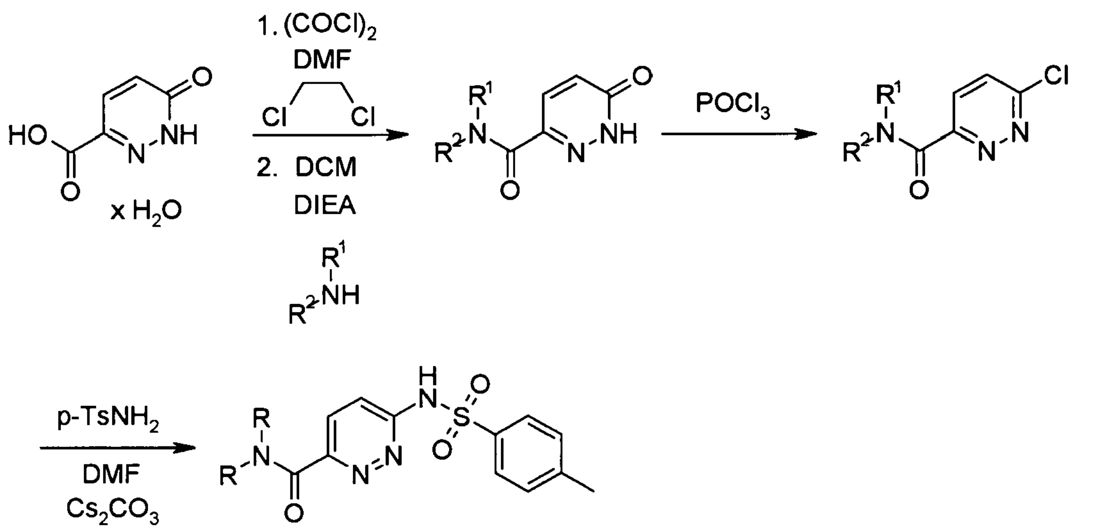


As depicted in Reaction Scheme 3, 6-oxo-1,6-dihydro-pyridazine-3-carboxylic acid can be reacted with a reagent such as oxalyl chloride in a suitable solvent like 1,2-dichloroethane in the presence of DMF to form the corresponding acid chloride which can subsequently be converted to the corresponding amide using an optionally substituted amine in the presence of a base such as DIEA in a suitable solvent such like DCM. The product of this reaction, optionally substituted 6-oxo-1,6-dihydro-pyridazine-3-carboxylic acid amide can be reacted with a reagent such as phosphorylchloride in a microwave reactor. Optionally substituted 6-chloro-pyridazine-3-carboxylic acid amide can be converted to the corresponding sulfonylamino-amide using p-toluenesulfonamide in the presence of a base such as cesium carbonate in a suitable solvent such as DMF under microwave irradiation.

## Reaction Scheme 4:

## Synthesis of imidazo[1,2-a]pyrimidines, -pyrazines and imidazo[1,2-b]pyridazines

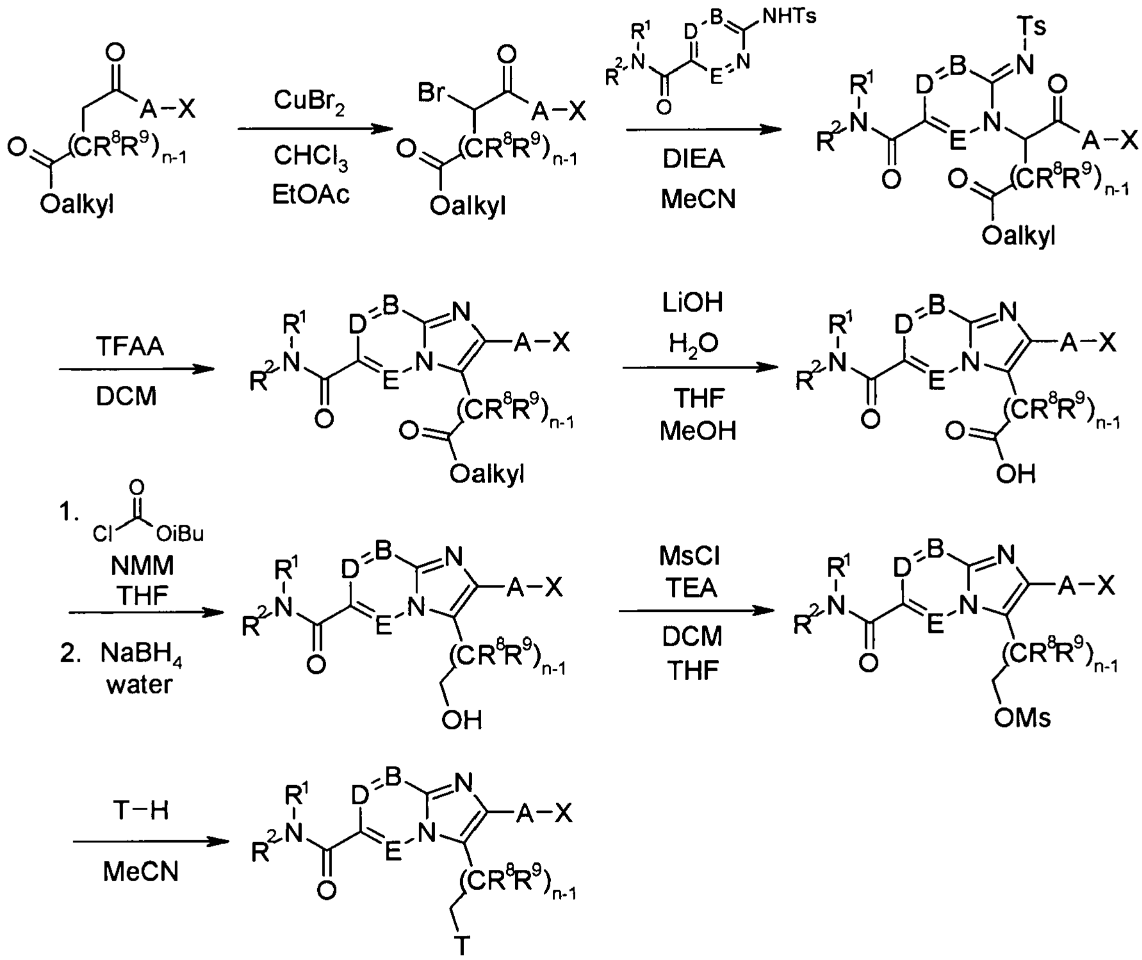


As shown in Reaction Scheme 4, optionally substituted ω-alkoxycarbonyl-α-bromoketones can be obtained from the corresponding ketone by reacting it for example with copper(II) bromide in a solvent such as mixture of ethyl acetate and chloroform at an appropriate temperature for a given time. The resulting α-bromoketones can then be reacted with sulfonylamino-amides in a solvent such as MeCN in the presence of an appropriate base, for example DIEA, to yield the N-alkylated sulfonylamino-amides. These intermediates can then be further cyclised to the corresponding imidazo[1,2-a]pyrimidines, -pyrazines and imidazo[1,2-b]pyridazines by treating them with TFAA in a suitable solvent such as DCM or 1,2-dichloroethane at an appropriate temperature for a given time. Ester function of optionally substituted imidazo[1,2-a]pyrimidines, -pyrazines and imidazo[1,2-b]pyridazines can be hydrolyzed under basic conditions using a reagent like lithium hydroxide monohydrate in a suitable solvent such as a mixture of water, THF and MeOH. The resulting acid can be activated with a reagent such as isobutyl chloroformate in the presence of a suitable base such as N-methylmorpholine in an appropriate solvent such as THF and subsequently be reduced to the corresponding alcohol with a reducing agent such as sodium borohydride in an appropriate solvent such as a mixture of THF and water. The alcohol function can be converted to a leaving group with a reagent such as mesyl chloride or tosyl chloride in an appropriate solvent such as mixture of DCM and THF in the presence of a suitable base like TEA. Product of this reaction can be treated with an amine T-H in an appropriate solvent like MeCN to yield the target molecule.

## Reaction Scheme 5:

## Synthesis of imidazo[1,2-a]pyrimidines, -pyrazines and imidazo[1,2-b]pyridazines

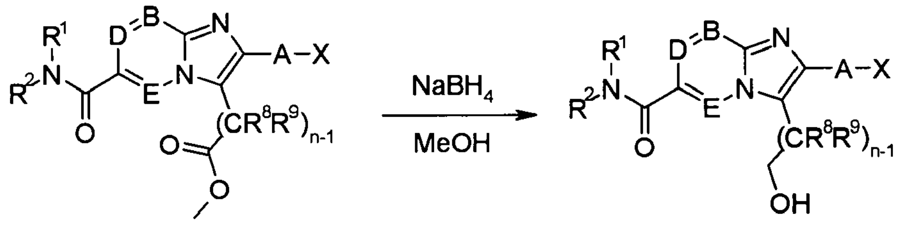


As shown in Reaction Scheme 5, methyl esters of optionally substituted imidazo[1,2-a]pyrimidines, -pyrazines and imidazo[1,2-b]pyridazines can be reduced to the corresponding alcohol with a reagent such as sodium borohydride in an appropriate solvent like methanol. The alcohol can be further reacted to the target molecules as depicted in Reaction Scheme 4.

## Reaction Scheme 6:

## Synthesis of imidazo[1,2-a]pyrimidines, -pyrazines and imidazo[1,2-b]pyridazines

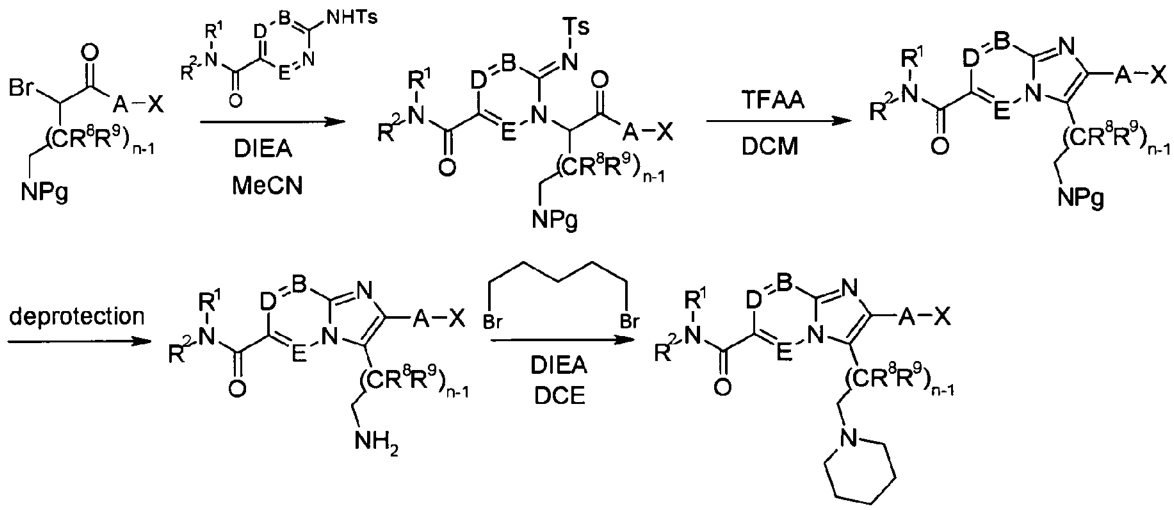


As shown in Reaction Scheme 6, optionally substituted N-protected ω-amino-α-bromoketones can be reacted with sulfonylamino-amides in a solvent such as MeCN in the presence of an appropriate base, for example DIEA, to yield the N-alkylated sulfonylamino-amides. These intermediates can then be further cyclised to the corresponding imidazo[1,2-a]pyrimidines, -pyrazines and imidazo[1,2-b]pyridazines by treating them with TFAA in a suitable solvent such as DCM or 1,2-dichloroethane at an appropriate temperature for a given time. The side chain amine function can be deprotected using a reagent such as TMSI in a suitable solvent such as MeCN in the case of a Z-protecting group. Phthalimids can be cleaved with hydrazine hydrate in an appropriate solvent like ethyl acetate. Optionally substituted imidazo[1,2-a]pyrimidines, -pyrazines and imidazo[1,2-b]pyridazines bearing the primary amino group in the side chain can be directly tested in the biologcial assay or are subjected to further derivatization. For example, reaction with 1,5-dibromopentane in an appropriate solvent like 1,2-dichloroethane in the presence of a suitable base such as DIEA leads to the corresponding piperidine derivatives.

## Reaction Scheme 7:

## Synthesis of imidazo[1,2-a]pyrimidines, -pyrazines and imidazo[1,2-b]pyridazines

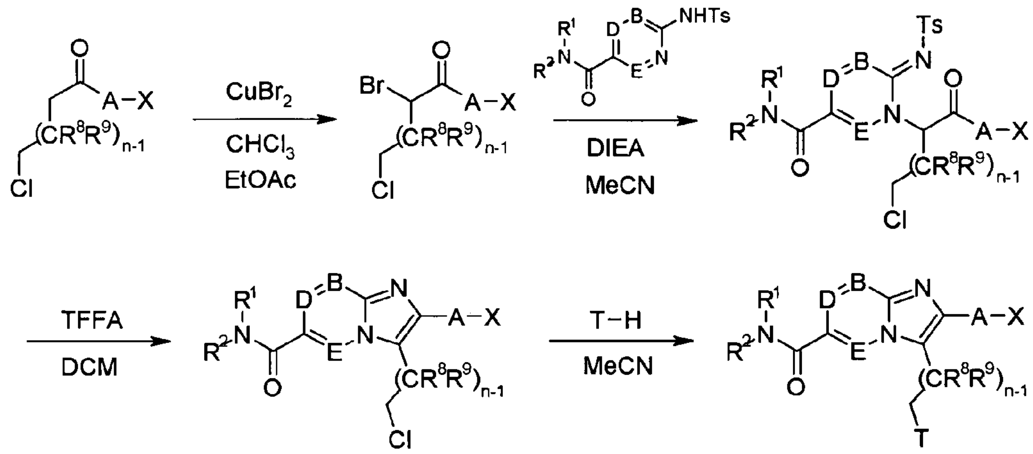


As shown in Reaction Scheme 7, optionally substituted ω-chloro-α-bromoketones can be obtained from the corresponding ketone by reacting it for example with copper (II) bromide in a solvent such as mixture of ethyl acetate and chloroform at an appropriate temperature for a given time. The resulting α-bromoketones can then be reacted with sulfonylamino-amides in a solvent such as MeCN in the presence of an appropriate base, for example DIEA, to yield the N-alkylated sulfonylamino-amides. These intermediates can then be further cyclised to the corresponding imidazo [1,2-a]pyrimidines, -pyrazines and imidazo[1,2-b]pyridazines by treating them with TFAA in a suitable solvent such as DCM or 1,2-dichloroethane at an appropriate temperature for a given time. The capping group T can be inserted by reacting the chloroalkyl substituted imidazo[1,2-a]pyrimidines, -pyrazines and imidazo[1,2-b]pyridazines with an capping group T-H in an appropriate solvent such as MeCN. When T-H is used in form of a hydrochloride, a suitable base such as DIEA is used in addition to liberate the free amine T-H.

## Reaction Scheme 8:

## Synthesis of imidazo[1,2-a]pyrimidines, -pyrazines and imidazo[1,2-b]pyridazines

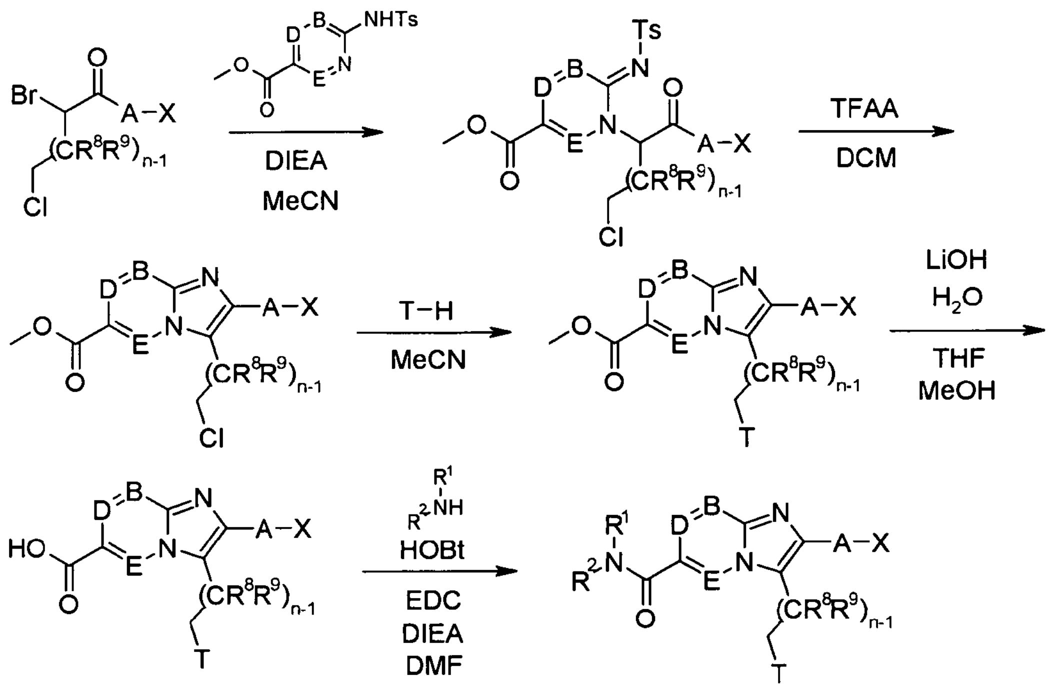


As shown in Reaction Scheme 8, optionally substituted ω-chloro-α-bromoketones can also be reacted with a sulfonylamino-ester in a solvent such as MeCN in the presence of an appropriate base, for example DIEA, to yield the N-alkylated sulfonylamino-esters. These intermediates can then be further cyclised to the corresponding imidazo[1,2-a]pyrimidines, -pyrazines and imidazo[1,2-b]pyridazines by treating them with TFAA in a suitable solvent such as DCM or 1,2-dichloroethane at an appropriate temperature for a given time. The capping group T can be inserted by reacting the chloroalkyl substituted imidazo[1,2-a]pyrimidines, -pyrazines and imidazo[1,2-b]pyridazines with a capping group T-H in an appropriate solvent such as MeCN. When T-H is used in form of a hydrochloride, a suitable base such as DIEA is used in addition to liberate the free amine T-H. Ester function of optionally substituted imidazo[1,2-a]pyrimidines, -pyrazines and imidazo[1,2-b]pyridazines can be hydrolyzed under basic conditions using a reagent like lithium hydroxide monohydrate in a suitable solvent such as a mixture of water, THF and MeOH. The product of the saponification can be isolated as lithium salt or as the corresponding acid. Alternatively, the ester function can also be cleaved under acidic conditions for example using a reagent such as aqueous hydrochloric acid. The product of the ester cleavage can be introduced into the next step as acid or lithium salt. Amide formation can be achieved using standard peptide coupling procedures. The acid can be coupled with an amine $HNR^1R^2$ in the presence of EDC/HOBt, a base such as diisopropylethylamine and a solvent such as dichloromethane. A suitable solvent, such as DCM, DMF, THF or a mixture of the above solvents, can be used for the coupling procedure. A suitable base includes triethylamine (TEA), diisopropylethylamine (DIEA), N-methylmorpholine (NMM), collidine or 2,6-lutidine. A base may not be needed when EDC/HOBt is used.

**Reaction Scheme 9:**

**Chloropyridine hydrolysis**

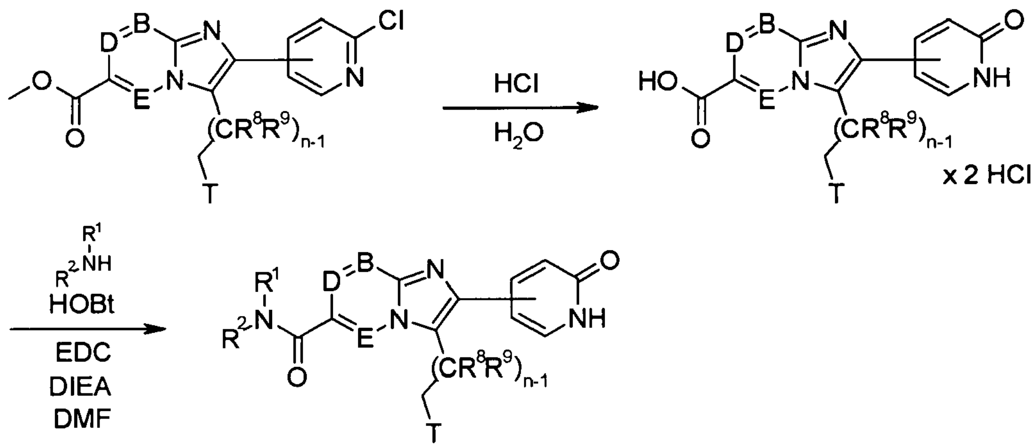


Optionally substituted imidazo[1,2-a]pyrimidines, -pyrazines and imidazo[1,2-b]pyridazines bearing a chloropyridine or bromopyridine as residue -A-X can be converted to the corresponding pyridones using a reagent such as aqueous hydrochloric acid at a suitable temperature as shown in Reaction Scheme 9. At the same time the ester function is also hydrolyzed. The acids can be coupled with amines $HNR^1R^2$ as described above.

**Reaction Scheme 10:**

**Synthesis of imidazo[1,2-a]pyrimidines and -pyrazines**

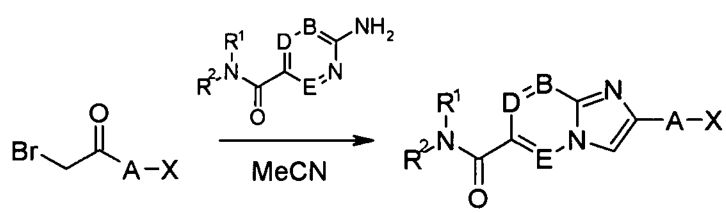


As shown in Reaction Scheme 10 optionally substituted aminopyrimidine- and aminopyrazine-amides, which can be obtained as shown in Reaction Scheme 1, can be converted to imidazo[1,2-a]pyrimidine and -pyrazine 6-carboxylic acid amides by reaction with α-bromoketones in a solvent like MeCN. This reaction can be carried out either in a flask in refluxing solvent or any other appropriate temperature or in a microwave reaction system. The reaction products can be purified by standard procedures or may precipitate directly from the solution upon cooling and may thus be used in subsequent reactions without further purification.

**Reaction Scheme 11:**

**Mannich reaction**

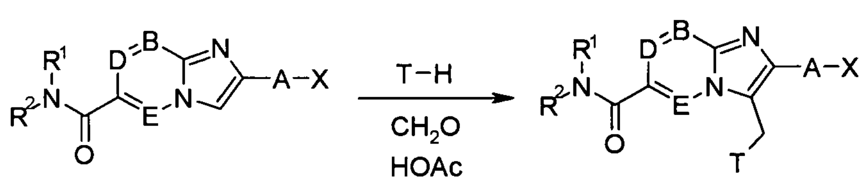

As shown in Reaction Scheme 11, products from Reaction Scheme 10 optionally substituted imidazo[1,2-a] pyrimidine and -pyrazine 6-carboxylic acid amides may be used in a Mannich-reaction to give 3-aminomethyl-imidazo [1,2-a] pyrimidine and -pyrazine 6-carboxylic acid amides by reacting the imidazo[1,2-a]pyrimidine and -pyrazine 6-carboxylic acid amides with an appropriate amine and aqueous formaldehyde solution in a solvent such as acetic acid. Diamines containing one nitrogen-protecting group can be further deprotected by treating the compound with an acid such as for example HCl in dioxane or TFA in DCM. Such compounds can then be purified by standard purification procedures such as flash chromatography or preparative HPLC.

## Reaction Scheme 12:

## Michael additions with α,β-unsaturated aldehydes

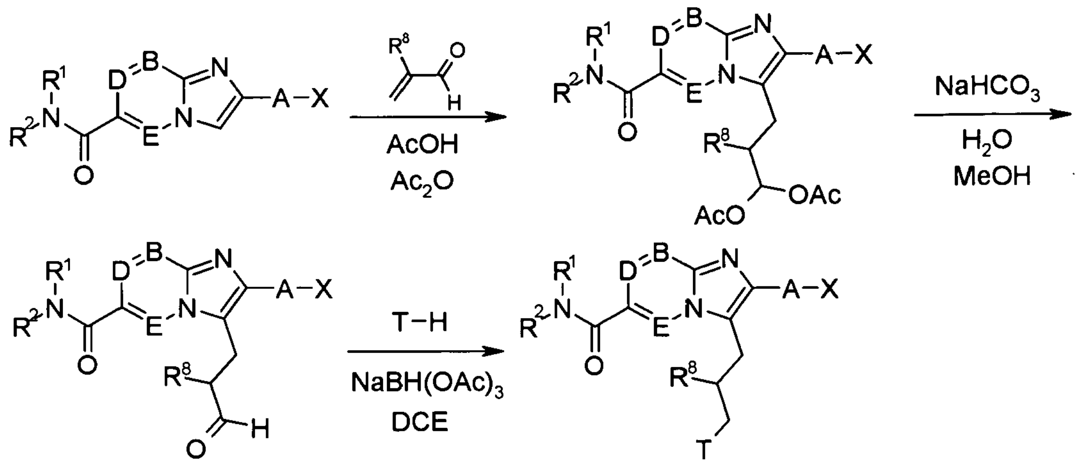


As depicted in Reaction Scheme 12, optionally substituted imidazo[1,2-a]pyrimidine and -pyrazine 6-carboxylic acid amides can be reacted in a Michael addition reaction with α,β-unsaturated aldehydes in a solvent such as a mixture of acetic acid and acetic anhydride at elevated temperature. The reaction may also be carried out in a microwave reactor. The product of this reaction can be treated with a base such as sodium bicarbonate in a suitable solvent like a mixture of water and methanol to yield the corresponding aldehydes which can be subjected to a reductive amination with an amine T-H in the presence of a reducing agent such as sodium triacetoxyborohydride in an appropriate solvent like DCE.

## Reaction Scheme 13:

## Michael additions with α,β-unsaturated ketones

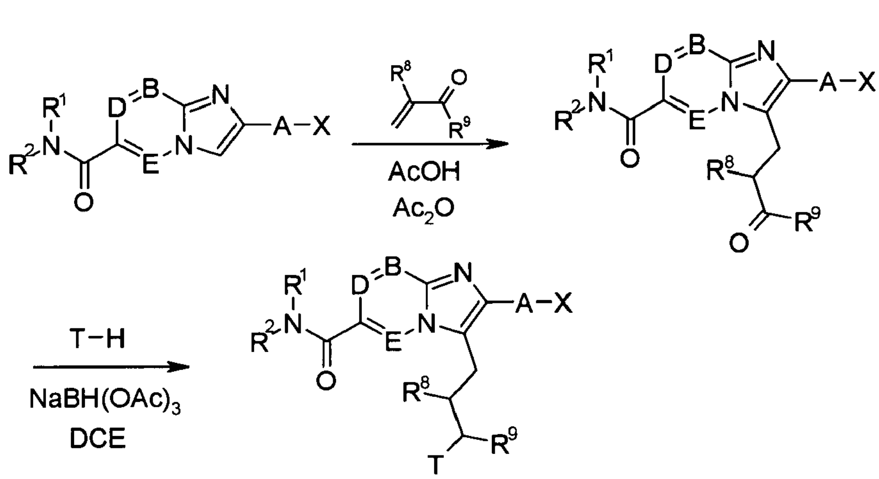

As shown in Reaction Scheme 13, Michael addition of optionally substituted imidazo[1,2-a]pyrimidine and -pyrazine 6-carboxylic acid amides can also be performed with $\alpha,\beta$-unsaturated ketones using the reaction conditions described in Reaction Scheme 12. In this case the product of the Michael addition reaction can be directly subjected to the reductive amination reaction.

## Reaction Scheme 14:

## Side chain alkylation

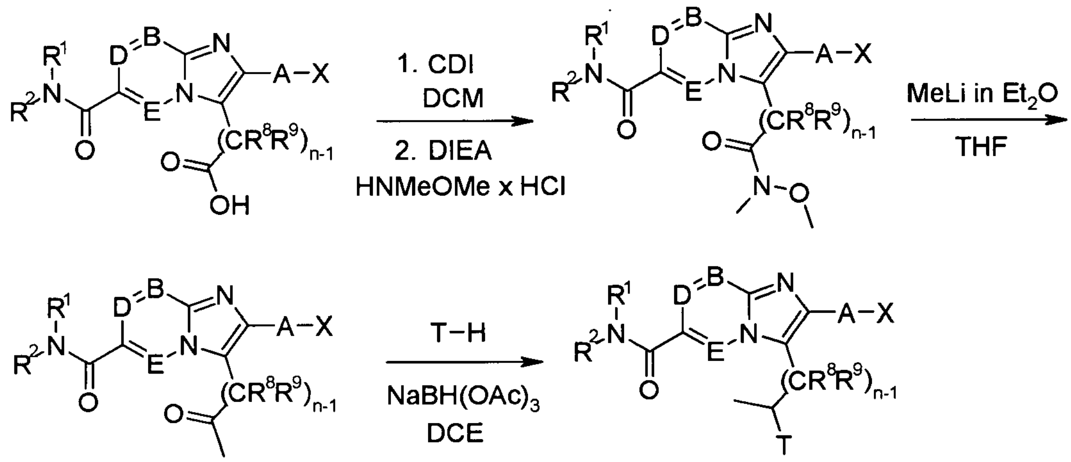


The products from Reaction Scheme 4, optionally substituted imidazo[1,2-a]pyrimidines, - pyrazines and imidazo[1,2-b]pyridazines bearing a carboxylate function in the side chain can be activated with a reagent such as CDI in an appropriate solvent like DCM and subsequently being reacted with O,N-dimethyl hydroxylamine hydrochloride in the presence of a suitable base such as DIEA. Reaction of the product with a reagent such as methyllithium in a suitable solvent such as THF or diethyl ether leads to the corrseponding ketones which can be reductively aminated with an amine T-H in the presence of a reducing agent such as sodium triacetoxyborohydride in an appropriate solvent like DCE.

**Analytical LC-MS**

The compounds of the present invention according to formula (I) were analyzed by analytical LC-MS. The conditions are summarized below.

<u>Analytical conditions summary:</u>

LC10Advp-Pump (Shimadzu) with SPD-M10Avp (Shimadzu) UV/Vis diode array detector and QP2010 MS-detector (Shimadzu) in ESI+ modus with UV-detection at 214, 254 and 275 nm,

Column: Waters XTerra MS C18, 3.5 $\mu$m, 2.1 * 100 mm, linear gradient with acetonitrile in water (0.15% HCOOH)

Flow rate of 0,4 ml/min;

| | |
|---|---|
| Mobile Phase A: | water (0.15% HCOOH) |
| Mobile Phase B: | acetonitrile (0.15% HCOOH) |

Methods are:

**A:**

start concentration 10% acetonitrile

10.00 B.Conc 60

11.00 B.Curve 2

12.00 B.Conc 99

15.00 B.Conc 99

15.20 B.Conc 10

18.00 Pump STOP

The following describes the detailed examples of the invention which can be prepared via the reaction schemes 1 to 14.

**Table 1:**

| | | | | | | | |
|---|---|---|---|---|---|---|---|
| 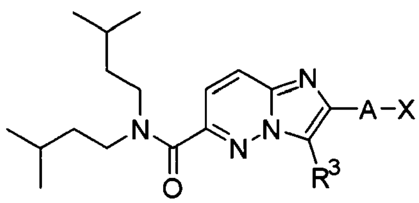 | | | | | | | |
| | | | | HPLC | | MS | |
| No. | salt | A-X | $R^3$ | $t_R$ (min) | method | MW (calc.) free base | $[M+H]^+$ (found) |
| 1 | HCOOH | 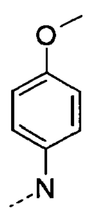 | 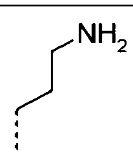 | 6.70 | A | 480.65 | 481 |
| 2 | HCl | 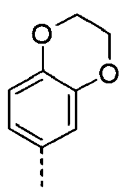 | 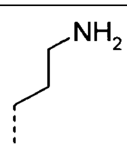 | 6.53 | A | 493.65 | 494 |
| 3 | HCl | 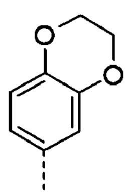 | 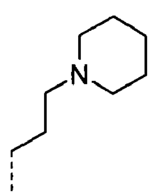 | 7.16 | A | 561.77 | 562 |

(continued)

| | | | | HPLC | | MS | |
|---|---|---|---|---|---|---|---|
| No. | salt | A-X | $R^3$ | $t_R$ (min) | method | MW (calc.) free base | $[M+H]^+$ (found) |
| 4 | - | 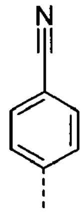 | 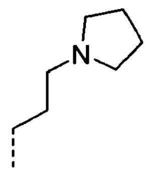 | 6.62 | A | 514.71 | 515 |

**Table 2:**

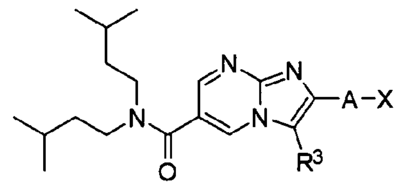

| | | | | HPLC | | MS | |
|---|---|---|---|---|---|---|---|
| No. | salt | A-X | $R^3$ | $t_R$ (min) | method | MW (calc.) free base | $[M+H]^+$ (found) |
| 5 | HCOOH | 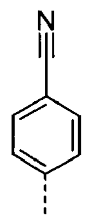 | 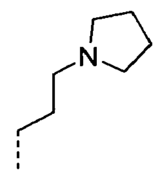 | 6.54 | A | 514.71 | 515 |
| 6 | - | 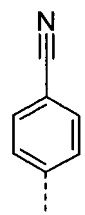 | 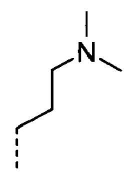 | 6.34 | A | 488.68 | 489 |

**Table 3**

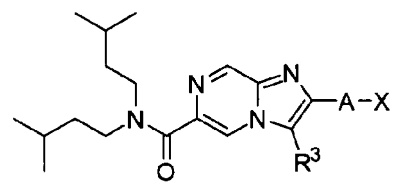

| | | | | HPLC | | MS | |
|---|---|---|---|---|---|---|---|
| No. | salt | A-X | $R^3$ | $t_R$ (min) | method | MW (calc.) free base free base | $[M+H]^+$ (found) |
| 7 | - | 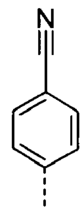 | 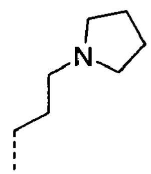 | 6.65 | A | 514.71 | 515 |
| 8 | HCOOH | 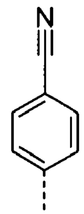 | 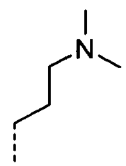 | 6.53 | A | 488.68 | 489 |
| 9 | HCOOH | 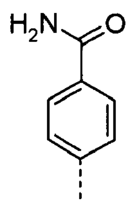 | 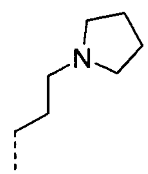 | 6.01 | A | 532.73 | 533 |

(continued)

| | | | | HPLC | | MS | |
|---|---|---|---|---|---|---|---|
| No. | salt | A-X | $R^3$ | $t_R$ (min) | method | MW (calc.) free base free base | $[M+H]^+$ (found) |
| 10 | HCOOH | 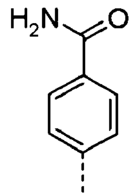 | 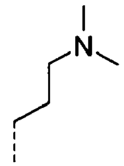 | 5.77 | A | 506.69 | 507 |

The following examples are provided to illustrate the invention and are not limiting the scope of the invention in any manner.

**Synthesis of Example 1:**

***Intermediate 1a):***

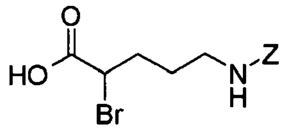

Hydrobromic acid (48% in water, 5.61 ml) and potassium bromide (4.96 g) were dissolved in water (60 ml). The solution was cooled to 0˚C and sodium nitrite (1.48 g) was added. HOrn(Z)-OH (3.00 g) was added in four portions and the reaction mixture was stirred at -3 to -5˚C for 2 h. The reaction mixture was extracted three times with ice cold ethyl acetate. The combined organic layer was washed twice with brine, dried over sodium sulfate and the solvent was removed under reduced pressure. The crude product was purified by flash chromatography.

***Intermediate 1b):***

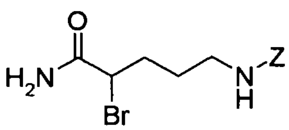

Rink amide resin (Novagel, 0.62 mmol/g, 1.45 g) was treated with DCM for 15 min. A solution of EDC (420 mg) in DCM (5 ml) was added followed by intermediate 1a) in DCM (5 ml). Additional DCM (10 ml) was added. The reaction mixture was shaken until testing with bromophenol blue indicated a full conversion. After 3 h the resin was filtered and washed with DCM (3 x). The product was cleaved from the resin by treatment with TFA/DCM 1:1. The resin was filtered off and washed three times with DCM. The filtrates were combined and the solvent was distilled off.

***Intermediate 1c):***

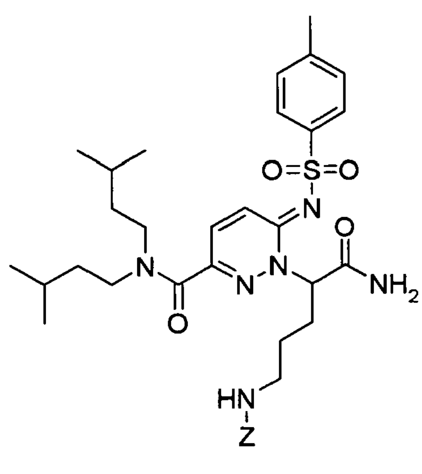

Intermediate 1b) (280 mg) was dissolved in DCM (15 ml) and DIEA (200 μl) was added. A solution of intermediate 4d) (328 mg) in DCM (15 ml) was added dropwise and the reaction mixture was stirred at room temperature overnight. LC-MS indicated no conversion. Therefore, more base was added and the mixture was reacted in a microwave reactor at 100˚C for 10 min. The solvent was switched to MeCN and the mixture was reacted in a microwave reactor at 120˚C for 110 min. The crude product was purified by flash chromatography.

***Intermediate 1d):***

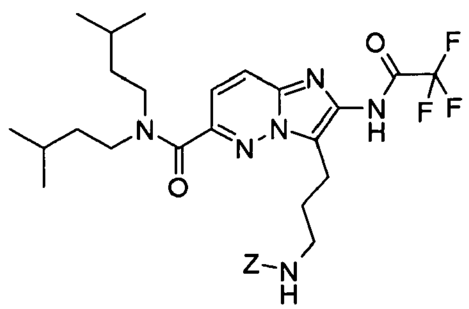

Intermediate 1c) (135 mg) was dissolved in DCM (2 ml) and TFAA (0.5 ml) was added. The mixture was stirred at room temperature for 20 min. The solvent was distilled off.

***Intermediate 1e):***

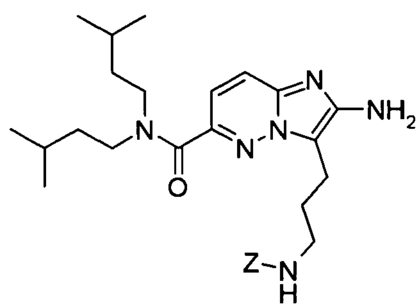

A solution of intermediate 1d) (180 mg) and lithium hydroxide hydrate (110 mg) in a mixture of THF and water (9:1) was heated to 140˚C in a microwave reactor for 20 min. The reaction mixture was diluted with buffer pH 7 and extracted with DCM. The organic layer was washed with brine, dried over sodium sulfate and the solvent was distilled off. The product was purified by flash chromatography.

***Intermediate 1f):***

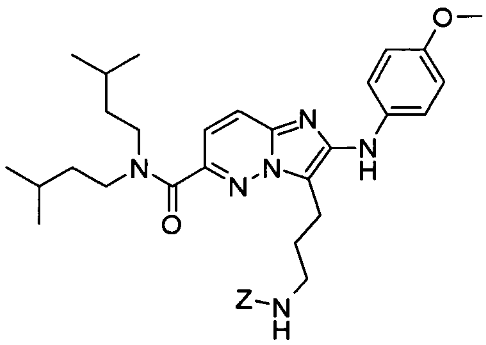

Intermediate 1e) (55 mg), 4-bromoanisol (14.3 μl), cesium carbonate (52 mg), $Pd_2(dba)_3$ (2.1 mg) and xantphos (4,5-bis(diphenylphosphino)-9,9-dimethylxanthene) (2.9 mg) were dissolved in 1,4-dioxane (1.0 ml). The reaction mixture was heated at 120°C in a microwave reactor for 30 min. LC-MS analysis indicated some conversion. The reaction mixture was heated at 120°C in a microwave reactor for 8 h. 4-Bromoanisol (14 μl) was added and the mixture was stirred at 130°C for 17 h. The reaction mixture was partitioned between DCM and buffer pH 7. The organic layer was washed with brine, dried over sodium sulfate and the solvent was distilled off. The product was purified by flash chromatography.

***Example 1:***

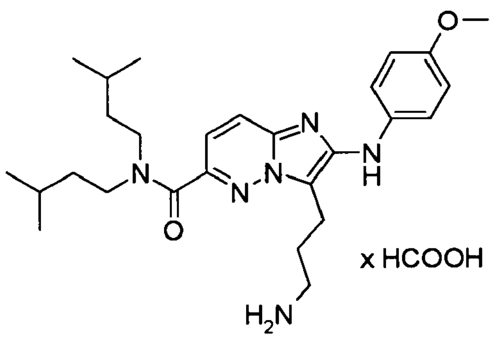

Intemediate 1f) (15 mg) was dissolved in dry acetonitrile (1 ml) under argon atmosphere and cooled to 0°C. TMSI (6 μl) was added and the reaction mixture was stirred at 0°C for 20 min. The reaction mixture was warmed to room temperature and TMSI (15 μl) was added. The reaction mixture was stirred at room temperature for 20 min. Methanol (1 ml) was added to stop the reaction and the solvents were removed under reduced pressure. The product was purified with preparative HPLC-MS.

**Synthesis of Example 2:**

***Intermediate 2a):***

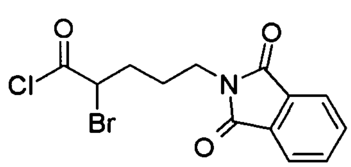

5-Phthalimido-2-bromovaleric acid (1.33 g) was dissolved in DCM (25 ml). DMF (20 μl) was added and the mixture was cooled to 0˚C. Oxalyl chloride (1.13 ml) was added; the mixture was warmed to room temperature and stirred for 2 h. The solvent was removed under reduced pressure.

***Intermediate 2b):***

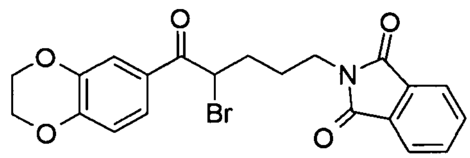

Intermediate 2a) (689 mg) was dissolved in 1,2-dichloroethane (5 ml) under argon atmosphere. At 0˚C $AlCl_3$ (1.06 g) was added followed by benzodioxane (262 μl). The reaction mixture was stirred at 0˚C for 25 min and poured on ice. The mixture was extracted with DCM. The organic layer was washed with brine, dried over sodium sulfate and the solvent was distilled off. The crude product was purified by column chromatography.

***Intermediate 2c):***

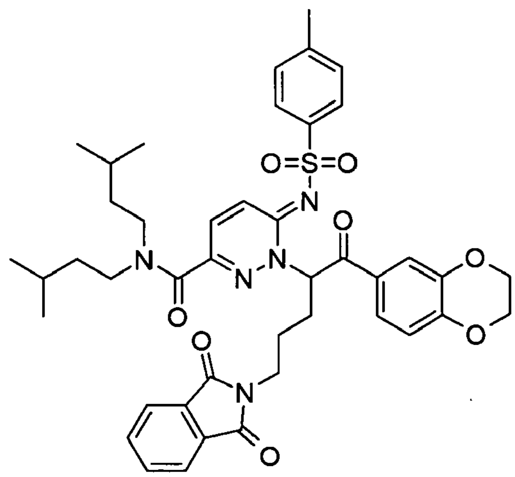

Intermediate 4d) (211 mg) and intermediate 2b) (260 mg) were dissolved in absolute MeCN (5 ml). DIEA (170 μl) was added and the reaction mixture was stirred at 120˚C for 45 min. The solvent was removed under reduced pressure and the crude product was purified by column chromatography.

***Example 2:***

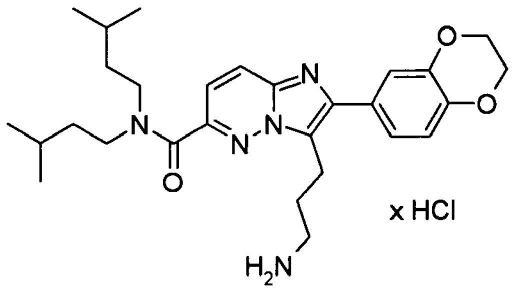

Intermediate 2c) (246 mg) was dissolved in a mixture of TFAA and DCM 3:10 (5 ml) and the solution was stirred at 70°C for 40 min and at 80°C overnight. The solvent was removed under reduced pressure and the crude product purified by column chromatography. The purified product was dissolved in ethyl acetate (4 ml) and hydrazine hydrate (200 µl) was added. The mixture was stirred at 50°C for 100 min and the solvent was removed under reduced pressure. The product was purified by column chromatography.
The pure product (5 mg) was dissolved in 4 N HCl in dioxane (1 ml) and the solvent was removed under reduced pressure.

**Synthesis of Example 3:**

***Example 3:***

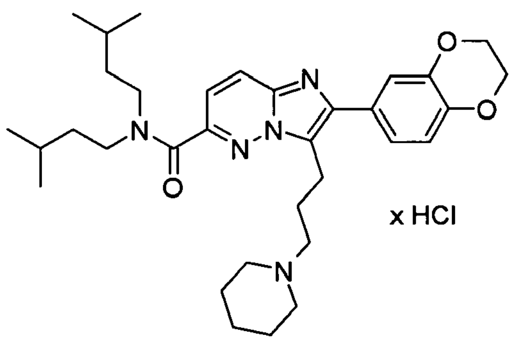

A mixture of Example 2 (71 mg, free base), 1,5-dibromopentane (59 µl) and DIEA (75 µl) in 1,2-dichloroethane (1 ml) was stirred at 90°C overnight. The reaction mixture was absorbed on silica gel and the product was purified by column chromatography. The pure product was dissolved in 4 N HCl in dioxane (2 ml) and lyophilized to yield a yellow solid.

**Synthesis of Example 4:**

***Intermediate 4a):***

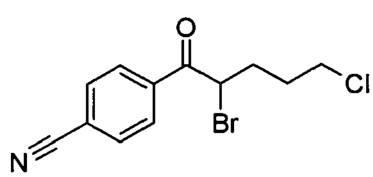

A solution of 5-chloro-1-(4-cyanophenyl)-1-oxopentane (1037 mg) in chloroform (20 ml) was added to a suspension of copper(II) bromide (1045 mg) in ethyl acetate (20 ml). The reaction mixture was heated to reflux. After 2 h a

second batch of copper(II) bromide (300 mg) was added and heating was continued for 3 h. TLC indicated that the reaction was not complete. A third batch of copper(II) bromide (300 mg) was added and heating was continued for 2 h. TLC indicated that the reaction was almost complete, traces of dibrominated compound were observed. The reaction mixture was filtered through Celite and the solvent was removed under reduced pressure. The crude product was purified by column chromatography.

***Intermediate 4b):***

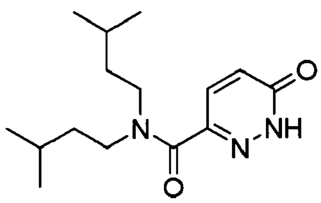

6-Oxo-1,6-dihydropyridazine-3-carboxylic acid monohydrate (2.0 g) and DMF (20 μl) were dissolved in 1,2-dichloroethane (20 ml) under argon and the reaction mixture was cooled to 0°C. Oxalyl chloride (2.7 ml) was added dropwise and the reaction mixture was stirred at room temperature overnight. The solvent was removed under reduced pressure. The residue was taken up in DCM (20 ml) and cooled to 0°C under argon. DIEA (3.3 ml) was added slowly followed by diisoamylamine (3.1 ml). The reaction mixture was stirred for 40 min. The reaction mixture was extracted twice with 5% citric acid, twice with saturated sodium bicarbonate solution and with brine. The organic phase was dried over sodium sulfate and the solvent was removed under reduced pressure.

***Intermediate 4c):***

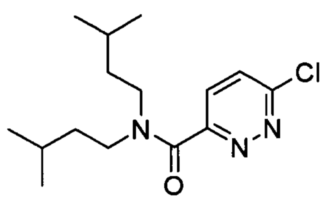

Intermediate 4b) (1.00 g) was dissolved in phosphorus oxychloride (4 ml). The solution was reacted in a microwave reactor at 100°C for 10 min. The reaction mixture was slowly poured on ice and the aqueous phase was extracted three times with DCM. The combined organic layer was washed with brine, dried over sodium sulfate and the solvent was removed under reduced pressure.

***Intermediate 4d):***

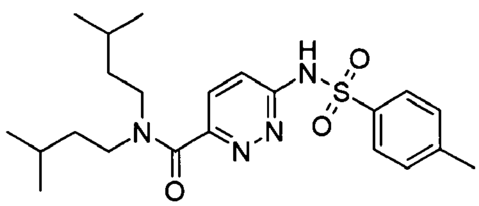

Intermediate 4c) (0.97 g) was dissolved in DMF (15 ml) and p-toluenesulfonamide (0.61 g) and cesium carbonate

(1.59 g) were added. The solution was reacted in a microwave reactor at 160°C for 30 min. The reaction mixture was poured in water and the aqueous phase was extracted three times with DCM. The combined organic layer was washed with brine, dried over sodium sulfate and the solvent was removed under reduced pressure.

***Intermediate 4e):***

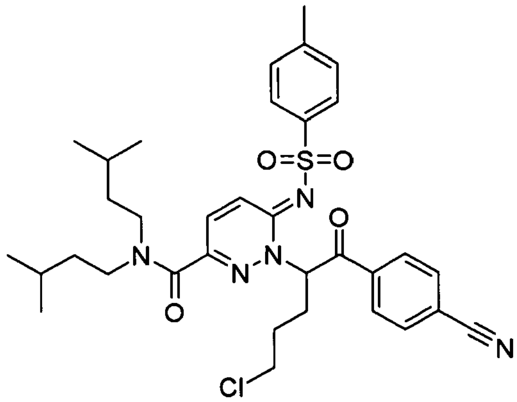

To a suspension of intermediate 4d) (216 mg) and ethyldiisopropylamine (192 μl) in acetonitrile (25 ml) was added intermediate 4a) (165 mg). The reaction mixture was heated to reflux for 3.5 h. At that time TLC indicated a full conversion. The reaction mixture was allowed to cool to room temperature and the solvent was removed under reduced pressure. The crude product was purified by flash chromatography to yield a pale yellow oil.

***Intermediate 4f):***

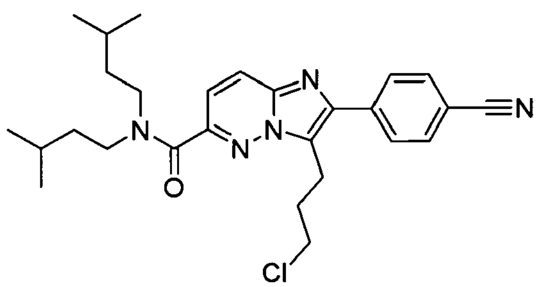

Intermediate 4e) (321 mg) was dissolved in 1,2-dichloroethane (18 ml). Trifluoroacetic acid anhydride (2 ml) was added and the reaction mixture was heated to reflux for 6 h. The solvent was removed under reduced pressure.

***Example 4:***

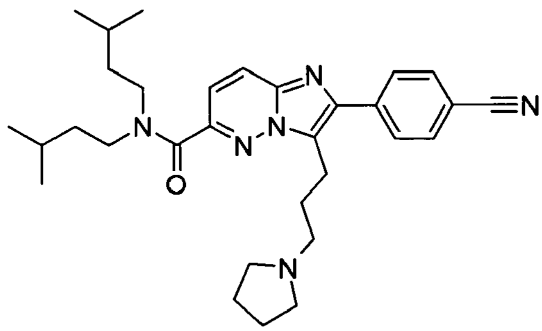

Intemediate 4f) (236 mg) was dissolved in acetonitrile (6 ml). Pyrrolidine (821 µl) was added and the reaction mixture was stirred at 70°C for 7 h. The solvent was removed under reduced pressure. The product was purified with preparative HPLC-MS. The purified product was dissolved in ethyl acetate (20 ml) and washed three times with 1 M NaOH and with brine. The organic phase was dried over sodium sulfate and the solvent was removed under reduced pressure. The product was obtained as yellow oil.

**Synthesis of Example 6:**

***Intermediate 6a):***

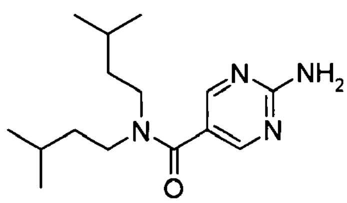

2-Aminopyrimidine-5-carboxylic acid (194 mg) was dissolved in DMF (15 ml) and then diisoamylamine (342 µl), EDC (320 mg), HOBt (256 mg) and finally DIEA (636 µl) were successively added. The reaction mixture was stirred at 50°C overnight. Volatiles were removed and then the residue was dissolved in of ethyl acetate (200 ml). The organic layer was washed with $NaHCO_3$ sat. (2 x 100 ml), then the combined aqueous layers were extracted back with ethyl acetate (100 ml). The combined organic layer was washed with brine, dried over $Na_2SO_4$, filtrated and the solvent was removed under reduced pressure.

***Intermediate 6b):***

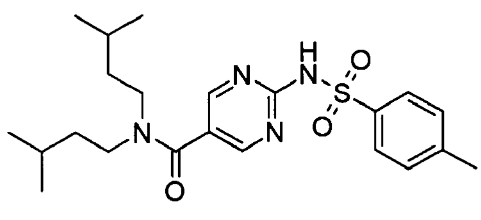

Intermediate 6a) (182 mg) was dissolved in pyridine (10 ml). p-Toluenesulfonylchloride (125 mg) was added and the reaction mixture was stirred at 85°C for 40 h. p-Toluenesulfonylchloride (250 mg) was added and the mixture was stirred at 130°C for 5 h. The solvent was removed under reduced pressure, the residue was suspended in water and the suspension stirred for 1 h. The mixture was extracted three times with ethyl acetate. The combined organic

layer was extracted twice with 1 M NaOH solution and with brine. The organic layer was dried over sodium sulfate and the solvent was removed under reduced pressure. The crude product was purified by column chromatography.

***Intermediate 6c):***

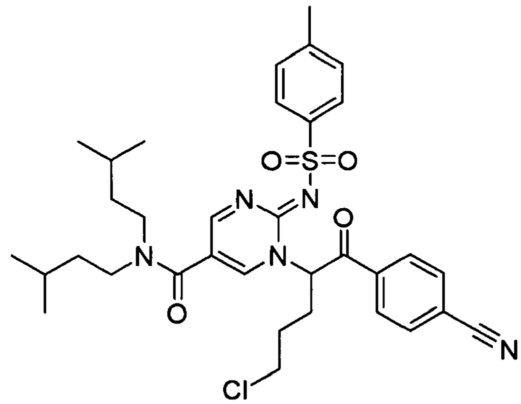

To a suspension of intermediate 6b) (32 mg) and ethyldiisopropylamine (26 μl) in acetonitrile (10 ml) was added intermediate 4a) (25 mg). The reaction mixture was heated to reflux for 3 h. The reaction mixture was allowed to cool to room temperature and the solvent was removed under reduced pressure. The crude product was purified by flash chromatography to yield a white solid.

***Intermediate 6d):***

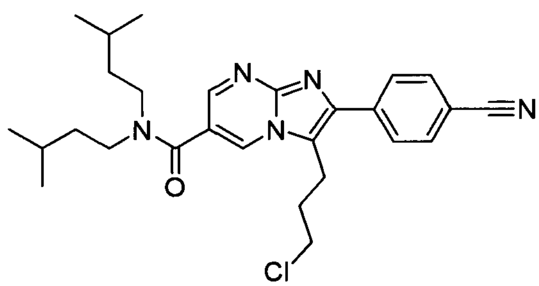

Intermediate 6c) (22 mg) was dissolved in 1,2-dichloroethane (5 ml). Trifluoroacetic acid anhydride (0.5 ml) was added and the reaction mixture was heated to 70˚C for 2 days. The solvent was removed under reduced pressure. The crude product was purified by column chromatography.

***Example 6:***

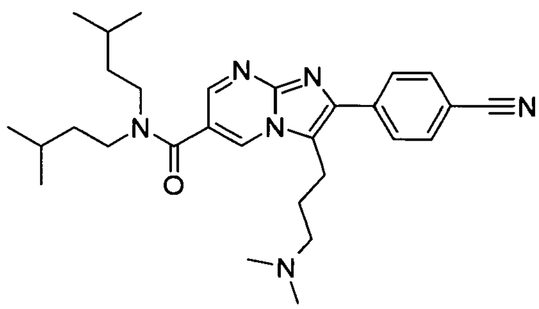

Intermediate 6d) (14 mg) was dissolved in acetonitrile (2.5 ml). A solution of 2 M dimethylamine in THF (290 µl) was added and the reaction mixture was stirred at 70˚C overnight. A second aliquot of 2 M dimethylamine in THF (145 µl) was added and the mixture was stirred at 70˚C for 2 days. The solvent was removed under reduced pressure. The product was purified with preparative HPLC-MS.

**Synthesis of Example 8:**

***Intermediate 8a):***

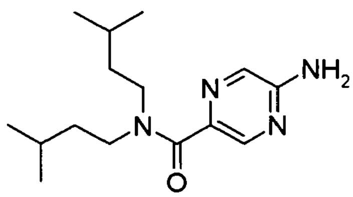

5-Aminopyrazine-2-carboxylic acid (500 mg) was dissolved in DMF (15 ml) and then diisoamylamine (885 µl), EDC (827 mg), HOBt (661 mg) and finally DIEA (751 µl) were successively added. The reaction mixture was stirred at 50˚C overnight. Volatiles were removed and the residue was dissolved in ethyl acetate. The organic layer was washed with brine, sat. $NaHCO_3$ and brine, dried over $Na_2SO_4$, filtrated and the solvent was removed under reduced pressure. The crude product was purified by column chromatography.

***Intermediate 8b):***

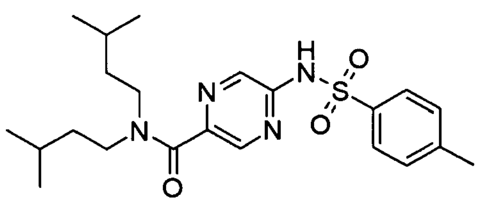

Intermediate 8a) (790 mg) was dissolved in pyridine (10 ml). p-Toluenesulfonylchloride (1353 mg) was added and the reaction mixture was stirred at 180˚C in a microwave reactor for 1 h. The solvent was removed under reduced pressure, the residue was suspended in water and the suspension stirred for 1 h. The mixture was extracted three times with ethyl acetate. The combined organic layer was extracted twice with 1 M NaOH solution and with brine. The organic layer was dried over sodium sulfate and the solvent was removed under reduced pressure. The crude product was purified by column chromatography.

*Intermediate 8c):*

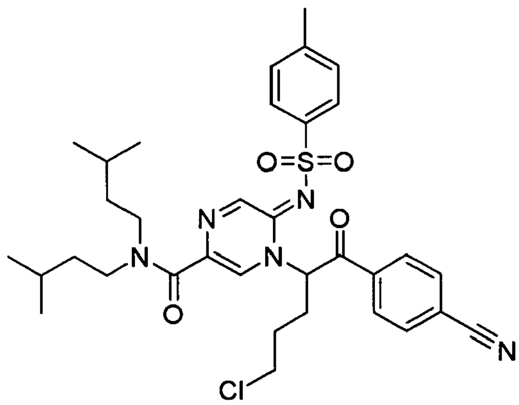

To a suspension of intermediate 8b) (800 mg) and ethyldiisopropylamine (709 µl) in acetonitrile (25 ml) was added intermediate 2a) (611 mg). The reaction mixture was heated to 50˚C for 20 h. The reaction mixture was allowed to cool to room temperature and the solvent was removed under reduced pressure. The crude product was purified by flash chromatography.

*Intermediate 8d):*

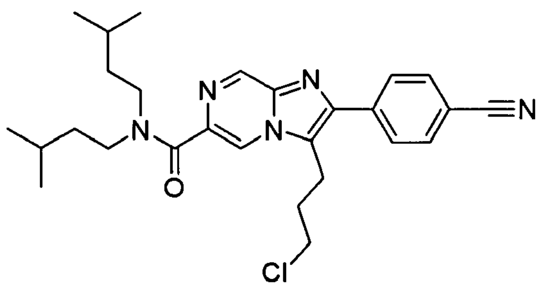

Intermediate 8c) (1.1 g) was dissolved in 1,2-dichloroethane (25 ml). Trifluoroacetic acid anhydride (5 ml) was added and the reaction mixture was stirred for 24 h. The solvent was removed under reduced pressure. The crude product was purified by column chromatography.

*Example 8:*

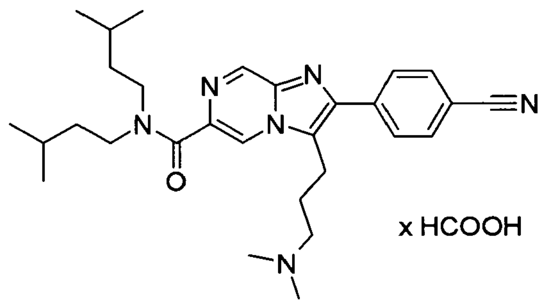

x HCOOH

Intemediate 8d) (120 mg) was dissolved in acetonitrile (5 ml). 2 M Dimethylamine in THF (1.25 ml) was added and the reaction mixture was stirred at 70˚C for 28 h. The solvent was removed under reduced pressure. The product was purified with preparative HPLC-MS.

**Synthesis of Example 10:**

***Example 10:***

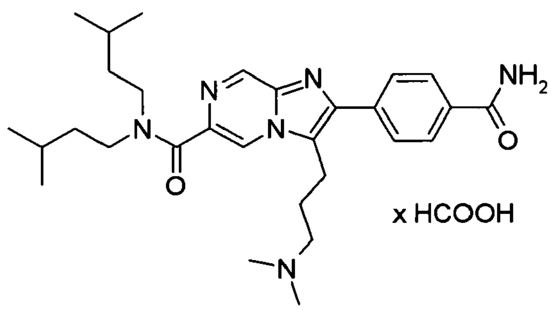

x HCOOH

To a suspension of example 8 (40 mg) in tert-butanol (3 ml) was added potassium hydroxide (25 mg) in form of a powder and the resulting mixture was heated to 70˚C for 4 h. The mixture was partitioned between ethyl acetate and brine. The aqueous layer was extracted twice with brine.
The solvent was removed under reduced pressure. The product was purified with preparative HPLC-MS.

**BIOLOGICAL ASSAYS**

**A. Binding Assay**

A membrane binding assay is used to identify competitive inhibitors of fluorescence labeled NDP-alpha-MSH binding to HEK293 cell membrane preparations expressing human melanocortin receptors.
The test compound or unlabeled NDP-alpha-MSH is dispensed at varying concentrations to a 384 well microtiter plate. Fluorescence labeled NDP-alpha-MSH is dispensed at a single concentration, followed by addition of membrane preparations. The plate is incubated for 5 h at room temperature.
The degree of fluorescence polarization is determined with a fluorescence polarization microplate reader.

**B. Functional Assay**

Agonistic activity of human melanocortin receptors is determined in a homogeneous membrane based assay. Competition between unlabeled cAMP and a fixed quantity of fluorescence labeled cAMP for a limited number of binding sites on a cAMP specific antibody is revealed by fluorescence polarization.
The test compound or unlabeled NDP-alpha-MSH is dispensed at varying concentrations to a 384 well microtiter plate. Membrane preparations from HEK293 cells expressing the human melanocortin receptors are added. After a short preincubation period, an appropriate amount of ATP, GTP and the cAMP antibody is added and the plate is further

incubated before the fluorescence labeled cAMP conjugate is dispensed. The plate is incubated for 2 h at 4˚C before it is read on a fluorescence polarization microplate reader. The amount of cAMP produced as a response to a test compound is compared to the production of cAMP resulting from stimulation with NDP-alpha-MSH.

Representative compounds of the present invention were tested and found to bind to the melanocortin-4 receptor. These compounds were generally found to have $IC_{50}$ values less than 2 $\mu$M.

**Table 4: Biological data for selected examples of the invention**

| Example | hMC-4R binding assay $IC_{50}/\mu M$ | hMC-4R functional assay $EC_{50}/\mu M$ | % activation functional assay |
|---|---|---|---|
| SHU9119 | 0.0019 | - | 7 |
| NDP-$\alpha$-MSH | 0.0011 | 3.4 | 100 |
| 1 | 0.49 | - | 0 |
| 2 | 1.1 | - | 0 |
| 3 | 0.12 | - | 0 |
| 4 | 0.30 | - | 0 |
| 5 | 0.18 | - | 0 |
| 6 | 1.00 | - | 0 |
| 7 | 0.020 | - | 0 |
| 8 | 0.19 | - | 0 |
| 9 | 0.048 | - | 0 |
| 10 | 0.28 | - | 0 |

## C. In Vivo Food Intake Models

### 1. Spontaneous Feeding Paradigm

Food intake in rats is measured after s.c., i.p. or p.o. administration of the test compound (see e.g. Chen, A.S. et al. Transgenic Res 2000 Apr;9(2):145-54).

### 2. Models of LPS-Induced Anorexia and Tumor-Induced Cachexia

Prevention or amelioration of anorexia induced by lipopolysaccharide (LPS) administration or cachexia induced by tumor growth is determined upon s.c., i.p. or p.o. administration of test compounds to rats (see e.g. Marks, D.L.; Ling, N and Cone, R.D. Cancer Res 2001 Feb 15;61(4):1432-8).

## D. In vitro ADME Assays

### 1. Microsomal Stability

### Experimental Procedure

Pooled human liver microsomes (pooled male and female) and pooled rat liver microsomes (male Sprague Dawley rats) are prepared. Microsomes are stored at -80˚C prior to use.

Microsomes (final concentration 0.5 mg/ml), 0.1 M phosphate buffer pH7.4 and test compound (final substrate concentration = 3 $\mu$M; final DMSO concentration = 0.25%) are pre-incubated at 37˚C prior to the addition of NADPH (final concentration = 1 mM) to initiate the reaction. The final incubation volume is 25 $\mu$l. A control incubation is included for each compound tested where 0.1 M phosphate buffer pH7.4 is added instead of NADPH (minus NADPH). Two control compounds are included with each species. All incubations are performed singularly for each test compound.

Each compound is incubated for 0, 5, 15, 30 and 45 min. The control (minus NADPH) is incubated for 45 min only. The reactions are stopped by the addition of 50 $\mu$l methanol containing internal standard at the appropriate time points. The incubation plates are centrifuged at 2,500 rpm for 20 min at 4˚C to precipitate the protein.

**Quantitative Analysis**

Following protein precipitation, the sample supernatants are combined in cassettes of up to 4 compounds and analysed using generic LC-MS/MS conditions.

**Data Analysis**

From a plot of the peak area ratio (compound peak area/internal standard peak area) against time, the gradient of the line is determined. Subsequently, half-life and intrinsic clearance are calculated using the equations below:

$$\text{Elimination rate constant (k)} = (\text{- gradient})$$

$$\text{Half life } (t_{1/2}) \text{ (min)} = \frac{0.693}{k}$$

$$\text{Intrinsic Clearance } (CL_{int}) \text{ (µl/min/mg protein)} = \frac{V \times 0.693}{t_{1/2}}$$

where V = Incubation volume µl/mg microsomal protein.

Two control compounds are included in the assay and if the values for these compounds are not within the specified limits the results are rejected and the experiment repeated.

**2. Hepatocyte Stability**

**Experimental Procedure**

Suspensions of cryopreserved hepatocytes are used for human hepatocyte stability assay (pooled from 3 individuals). All cryopreserved hepatocytes are purchased from In Vitro Technologies, Xenotech or TCS.
Incubations are performed at a test or control compound concentration of 3 µM at a cell density of $0.5 \times 10^6$ viable cells/mlL. The final DMSO concentration in the incubation is 0.25%. Control incubations are also performed in the absence of cells to reveal any non-enzymatic degradation.
Duplicate samples (50 µl) are removed from the incubation mixture at 0, 5, 10, 20, 40 and 60 min (control sample at 60 min only) and added to methanol, containing internal standard (100 µl), to stop the reaction.
Tolbutamide, 7-hydroxycoumarin, and testosterone are used as control compounds.
The samples are centrifuged (2500 rpm at 4°C for 20 min) and the supernatants at each time point are pooled for cassette analysis by LC-MS/MS using generic methods.

**Data Analysis**

From a plot of In peak area ratio (compound peak area/internal standard peak area) against time, the gradient of the line is determined. Subsequently, half-life and intrinsic clearance are calculated using the equations below:

$$\text{Elimination rate constant (k)} = (\text{- gradient})$$

$$\text{Half life } (t_{1/2}) \text{(min)} = \frac{0.693}{k}$$

$$\text{Intrinsic Clearance } (CL_{int})(\mu l/min/million\ cells) = \frac{V \times 0.693}{t_{1/2}}$$

where V = Incubation volume (μl)/number of cells

**3. Caco-2 Permeability (Bi-directional)**

**Experimental Procedure**

Caco-2 cells obtained from the ATCC at passage number 27 are used. Cells (passage number 40-60) are seeded on to Millipore Multiscreen Caco-2 plates at 1 x 105 cells/cm$^2$. They are cultured for 20 days in DMEM and media is changed every two or three days. On day 20 the permeability study is performed.
Hanks Balanced Salt Solution (HBSS) pH7.4 buffer with 25 mM HEPES and 10 mM glucose at 37°C is used as the medium in permeability studies. Incubations are carried out in an atmosphere of 5% $CO_2$ with a relative humidity of 95%. On day 20, the monolayers are prepared by rinsing both basolateral and apical surfaces twice with HBSS at 37°C. Cells are then incubated with HBSS in both apical and basolateral compartments for 40 min to stabilize physiological parameters.
HBSS is then removed from the apical compartment and replaced with test compound dosing solutions. The solutions are made by diluting 10 mM test compound in DMSO with HBSS to give a final test compound concentration of 10 μM (final DMSO concentration 1 %). The fluorescent integrity marker lucifer yellow is also included in the dosing solution. Analytical standards are made from dosing solutions. Test compound permeability is assessed in duplicate. On each plate compounds of known permeability characteristics are run as controls.
The apical compartment inserts are then placed into 'companion' plates containing fresh HBSS. For basolateral to apical (B-A) permeability determination the experiment is initiated by replacing buffer in the inserts then placing them in companion plates containing dosing solutions. At 120 min the companion plate is removed and apical and basolateral samples diluted for analysis by LC-MS/MS. The starting concentration ($C_0$) and experimental recovery is calculated from both apical and basolateral compartment concentrations.
The integrity of the monolayers throughout the experiment is checked by monitoring lucifer yellow permeation using fluorimetric analysis. Lucifer yellow permeation is low if monolayers have not been damaged. Test and control compounds are quantified by LC-MS/MS cassette analysis using a 5-point calibration with appropriate dilution of the samples. Generic analytical conditions are used.

If a lucifer yellow $P_{app}$ value is above QC limits in one individual test compound well, then an n=1 result is reported. If lucifer yellow $P_{app}$ values are above QC limits in both replicate wells for a test compound, the compound is re-tested. Consistently high lucifer yellow permeation for a particular compound in both wells indicates toxicity. No further experiments are performed in this case.

**Data Analysis**

The permeability coefficient for each compound ($P_{app}$) is calculated from the following equation:

$$P_{app} = \frac{dQ/dt}{C_0 \times A}$$

Where dQ/dt is the rate of permeation of the drug across the cells, $C_0$ is the donor compartment concentration at time zero and A is the area of the cell monolayer. $C_0$ is obtained from analysis of donor and receiver compartments at the end of the incubation period. It is assumed that all of the test compound measured after 120 min incubation was initially present in the donor compartment at 0 min. An asymmetry index (AI) is derived as follows:

$$AI = \frac{P_{app}\,(B\text{-}A)}{P_{app}\,(A\text{-}B)}$$

An asymmetry index above unity shows efflux from the Caco-2 cells, which indicates that the compound may have potential absorption problems in vivo.

The apparent permeability ($P_{app}$ (A-B)) values of test compounds are compared to those of control compounds, atenolol and propranolol, that have human absorption of approximately 50 and 90% respectively (Zhao, Y.H., et al., (2001). Evaluation of Human Intestinal Absorption Data and Subsequent Derivation of a Quantitative Structure-Activity Relationship (QSAR) with the Abraham Descriptors. Journal of Pharmaceutical Sciences. 90 (6), 749-784). Talinolol (a known P-gp substrate (Deferme, S., Mols, R., Van Driessche, W., Augustijns, P. (2002). Apricot Extract Inhibits the P-gp-Mediated Efflux of Talinolol. Journal of Pharmaceutical Sciences. 91 (12), 2539-48)) is also included as a control compound to assess whether functional P-gp is present in the Caco-2 cell monolayer.

**4. Cytochrome P450 Inhibition (5 Isoform $IC_{50}$ Determination))**

**Experimental Procedure**

**CYP1A Inhibition**

Six test compound concentrations (0.05, 0.25, 0.5, 2.5, 5, 25 μM in DMSO; final DMSO concentration = 0.35%) are incubated with human liver microsomes (0.25 mg/ml) and NADPH (1 mM) in the presence of the probe substrate ethoxyresorufin (0.5 μM) for 5 min at 37°C. The selective CYP1A inhibitor, alpha-naphthoflavone, is screened alongside the test compounds as a positive control.

**CYP2C9 Inhibition**

Six test compound concentrations (0.05, 0.25, 0.5, 2.5, 5, 25 μM in DMSO; final DMSO concentration = 0.25%) are incubated with human liver microsomes (1 mg/ml) and NADPH (1 mM) in the presence of the probe substrate tolbutamide (120 μM) for 60 min at 37°C. The selective CYP2C9 inhibitor, sulphaphenazole, is screened alongside the test compounds as a positive control.

**CYP2C19 Inhibition**

Six test compound concentrations (0.05, 0.25, 0.5, 2.5, 5, 25 μM in DMSO; final DMSO concentration = 0.25%) are incubated with human liver microsomes (0.5 mg/ml) and NADPH (1 mM) in the presence of the probe substrate mephenytoin (25 μM) for 60 min at 37°C. The selective CYP2C19 inhibitor, tranylcypromine, is screened alongside the test compounds as a positive control.

**CYP2D6 Inhibition**

Six test compound concentrations (0.05, 0.25, 0.5, 2.5, 5, 25 μM in DMSO; final DMSO concentration = 0.25%) are incubated with human liver microsomes (0.5 mg/ml) and NADPH (1 mM) in the presence of the probe substrate dextromethorphane (5 μM) for 30 min at 37°C. The selective CYP2D6 inhibitor, quinidine, is screened alongside the test compounds as a positive control.

**CYP3A4 Inhibition**

Six test compound concentrations (0.05, 0.25, 0.5, 2.5, 5, 25 μM in DMSO; final DMSO concentration 0.26%) are incubated with human liver microsomes (0.25 mg/ml) and NADPH (1 mM) in the presence of the probe substrate midazolam (2.5 μM) for 5 min at 37°C. The selective CYP3A4 inhibitor, ketoconazole, is screened alongside the test compounds as a positive control.

For the CYP1A incubations, the reactions are terminated by the addition of methanol, and the formation of the metabolite, resorufin, is monitored by fluorescence (excitation wavelength = 535 nm, emission wavelength = 595 nm). For the CYP2C9, CYP2C19, CYP2D6, and CYP3A4 incubations, the reactions are terminated by the addition of methanol containing internal standard. The samples are then centrifuged, and the supernatants are combined, for the simultaneous

analysis of 4-hydroxytolbutamide, 4-hydroxymephenytoin, dextrorphan, and 1-hydroxymidazolam plus internal standard by LC-MS/MS. Generic LC-MS/MS conditions are used. Formic acid in deionised water (final concentration = 0.1%) is added to the final sample prior to analysis. A decrease in the formation of the metabolites compared to vehicle control is used to calculate an $IC_{50}$ value (test compound concentration which produces 50% inhibition).

**5. Plasma Protein Binding (10%)**

**Experimental Procedure**

Solutions of test compound (5 μM, 0.5% final DMSO concentration) are prepared in buffer (pH 7.4) and 10% plasma (v/v in buffer). The experiment is performed using equilibrium dialysis with the two compartments separated by a semi-permeable membrane. The buffer solution is added to one side of the membrane and the plasma solution to the other side. Standards are prepared in plasma and buffer and are incubated at 37°C. Corresponding solutions for each compound are analyzed in cassettes by LC-MS/MS.

**Quantitative Analysis**

After equilibration, samples are taken from both sides of the membrane. The solutions for each batch of compounds are combined into two groups (plasma-free and plasma-containing) then cassette analyzed by LC-MS/MS using two sets of calibration standards for plasma-free (7 points) and plasma-containing solutions (6 points). Generic LC-MS/MS conditions are used. Samples are quantified using standard curves prepared in the equivalent matrix. The compounds are tested in duplicate.
A control compound is included in each experiment.

**Data Analysis**

$$fu = \frac{1 - ((PC - PF))}{(PC)}$$

fu = fraction unbound
PC = sample concentration in protein containing side
PF = sample concentration in protein free side
fu at 10% plasma is converted to fu 100% plasma using the following equation:

$$fu_{100\%} = \frac{fu_{10\%}}{10-(9^{*}fu_{10\%})}$$

<u>**Examples of a Pharmaceutical Composition**</u>

As a specific embodiment of an oral composition of a compound of the present invention, 34 mg of Example 5 is formulated with sufficient finely divided lactose to provide a total amount of 580 to 590 mg to fill a size 0 hard gelatin capsule.

As another specific embodiment of an oral composition of a compound of the present invention, 36 mg of Example 7 is formulated with sufficient finely divided lactose to provide a total amount of 580 to 590 mg to fill a size 0 hard gelatin capsule.

While the invention has been described and illustrated in reference to certain preferred embodiments thereof, those skilled in the art will appreciate that various changes, modifications and substitutions can be made therein without departing from the spirit and scope of the invention. For example, effective dosages, other than the preferred doses as set forth above, may be applicable as a consequence of the specific pharmacological responses observed and may vary depending upon the particular active compound selected, as well as from the type of formulation and mode of

administration employed, and such expected variations or differences in the results are contemplated in accordance with the objects and practices of the present invention. It is intended, therefore, that the invention be limited only by the scope of the claims which follow and that such claims be interpreted as broadly as is reasonable.

## Claims

**1.** A compound according to formula (I)

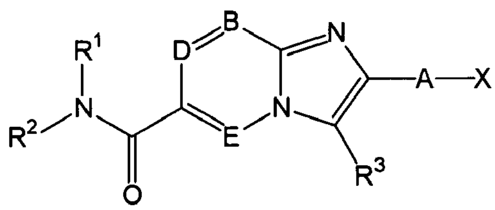

(I)

and enantiomers, diastereomers, tautomers, solvates and pharmaceutically acceptable salts thereof,
wherein
$R^1$ and $R^2$ are independently from each other selected from

H,
$C_{1-6}$alkyl,
$C_{1-6}$alkylene-O-$C_{1-6}$alkyl
$C_{1-3}$alkylene-heterocyclyl, and
$C_{1-6}$alkylene-$C_{3-7}$cycloalkyl, or

$R^1$ and $R^2$, together with the nitrogen atom to which they are attached to, form a 5 to 6-membered ring which may additionally contain one oxygen atom in the ring and which is unsubstituted or substituted by one or more substituents selected from OH, $C_{1-6}$alkyl, O-$C_{1-6}$alkyl, $C_{0-3}$alkylene-$C_{3-5}$cycloalkyl, $C_{1-6}$alkylene-O-$C_{1-6}$alkyl and $(CH_2)_{0-3}$phenyl;
B, D and E are independently from each other selected from CH and N with the proviso that one of B, D and E represents N;
A is -NH-,

-$C_{1-6}$alkylene,
-$C_{2-6}$alkenylene,
-$C_{2-6}$alkinylene or
a bond
wherein alkylene, alkenylene and alkinylene are unsubstituted or substituted with one ore more $R^7$;

$R^7$ is independently selected from

$C_{1-6}$alkyl,
$OR^{14}$,
$NR^{15a}R^{15b}$,
halogen,
phenyl and
heteroaryl,
wherein phenyl and heteroaryl are unsubstituted or substituted by 1 to 3 $R^{4a}$;

X is H,
CN,
$C_{3-8}$cycloalkyl, unsubstituted or substituted with one or more halogen atoms, phenyl,
phenyl which is fused with a saturated heterocyclic 6-membered ring,
wherein the heterocyclic ring contains 1 or 2 heteroatoms independently selected from O and N,
4 to 8-membered saturated or unsaturated heterocyclyl containing 1 to 4 heteroatoms independently selected from N, O and S,

5- to 6-membered heteroaryl containing 1 to 4 heteroatoms independently selected from N, O and S,
$-C(O)-R^6$
$-OR^{14}$,
halogen or
$NR^{15a}R^{15b}$
wherein each phenyl, heterocyclyl or heteroaryl is unsubstituted or substituted by 1 to 3 $R^{4a}$ and/or 1 $R^{4b}$ and/or 1 $R^5$;
$R^{4a}$ is halogen,
CN,
$C_{1-6}$alkyl, unsubstituted or substituted with one or more substituents independently selected from halogen, OH and $O-C_{1-6}$alkyl,
$O-C_{1-6}$alkyl, wherein alkyl is unsubstituted or substituted with one or more halogen atoms, or
$R^{4b}$ is $C(O)NH_2$,

$C(O)NH-C_{1-6}$alkyl,
$C(O)N-(C_{1-6}alkyl)_2$,
$SO_2-C_{1-6}$alkyl,
$C(O)NH-SO_2-C_{1-6}$alkyl,
oxo, whereby the ring is at least partially saturated,
$NH_2$,
$NH-C_{1-6}$alkyl,
$N-(C_{1-6}alkyl)_2$,
$NH-SO_2-CH_3$, or
$NH-SO_2-CF_3$;

$R^5$ is 5 to 6-membered saturated or unsaturated heterocyclyl containing 1 to 3 heteroatoms independently selected from N, O and S or
5 to 6-membered heteroaryl containing 1 to 3 heteroatoms independently selected from N, O and S
wherein each heterocyclyl and heteroaryl is unsubstituted or substituted by 1 Or 2 $R^{4a}$;
$R^6$ is H,
OH,
$C_{1-6}$alkyl, unsubstituted or substituted with one or more halogen atoms,
$O-C_{1-6}$alkyl, wherein alkyl is unsubstituted or substituted with one or more $R^{16}$,
phenyl,
4 to 8-membered saturated or unsaturated heterocyclyl containing 1 to 3 heteroatoms selected from N, O or S or
$NR^{16a}R^{16b}$,
wherein each phenyl and heterocyclyl is unsubstituted or substituted by 1 to 3 $R^{4a}$ and/or 1 $R^5$;
$R^3$ is $-(CR^8R^9)_n-T$;
$R^8$ and $R^9$ are independently from each other selected from

H,
OH,
halogen,
$C_{1-6}$alkyl, and
$O-C_{1-6}$alkyl,

n is 1 to 6;
T is

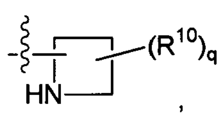
,
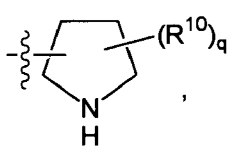
,
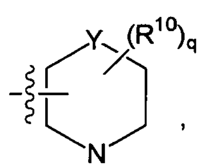
,

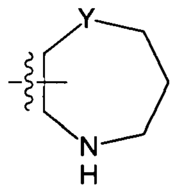 , 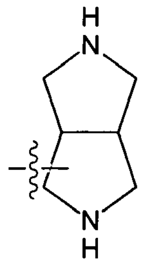 , 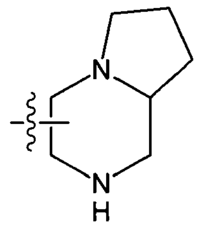

or $NR^{12}R^{13}$;
$R^{10}$ is H,

- $NH_2$,
- OH,
- O-$C_{1-6}$alkyl,

wherein alkyl is unsubstituted or substituted by 1 to 3 halogenatoms

- $C_{1-6}$alkyl,
- halogen,
- NH($C_{1-6}$alkyl),
- N($C_{1-6}$alkyl)$_2$,
- phenyl or
- heteroaryl,

wherein phenyl and heteroaryl are unsubstituted or substituted by 1 to 3 $R^{4a}$;
q is 1 or 2;
Y is $CH_2$,
$NR^{11}$ or
O;
$R^{11}$ is H,

- $C_1$-$_6$alkylor
- $(CH_2)_{0-6}$-$C_{3-7}$cycloalkyl;

$R^{12}$ and $R^{13}$ are independently from each other selected from

- H,
- $C_{1-6}$alkyl,
- $(CH_2)_{0-2}$-$C_{3-7}$cycloalkyl and
- $C_{1-6}$alkylene-O-$C_{1-6}$alkyl;

wherein alkyl, alkylene and cycloalkyl are unsubstituted or substituted by 1 to 3 $R^{4a}$;
$R^{14}$ is H,

- $C_1$-$_6$alkyl, unsubstituted or substituted with one or more substituents selected from halogen,
- phenyl or
- heteroaryl,

wherein phenyl and heteroaryl are unsubstituted or substituted by 1 to 3 $R^{4a}$;
$R^{15a}$ and $R^{15b}$ are independently from each other selected from

- H,
- $C_{1-6}$alkyl, unsubstituted or substituted with one or more substituents selected from halogen, OH, O($C_{1-6}$alkyl), $NH_2$, NH($C_{1-6}$alkyl) and N($C_{1-6}$alkyl)$_2$,

phenyl and
heteroaryl,

wherein phenyl and heteroaryl are unsubstituted or substituted by 1 to 3 $R^{4a}$, and
$C(O)C_{1-6}$alkyl;
$R^{16}$, $R^{16a}$ and $R^{16b}$ are independently from each other selected from

H,
$C_{1-6}$alkyl, unsubstituted or substituted with one or more substituents selected from halogen, OH, $O(C_{1-6}$alkyl), $NH_2$, $NH(C_{1-6}$alkyl) and $N(C_{1-6}$alkyl$)_2$,
$C_{0-3}$alkylene-$C_{3-5}$Cycloalkyl,
phenyl and
heteroaryl,

wherein phenyl and heteroaryl are unsubstituted or substituted by 1 to 3 $R^{4a}$.

**2.** The compound of claim 1 wherein A is -NH- or a bond.

**3.** The compound of claim 1 or 2 wherein $R^1$ and $R^2$ are independently from each other $C_{3-6}$alkyl or $R^1$ and $R^2$ form together with the nitrogen atom to which they are attached to a 5 to 6-membered ring which may additionally contain one oxygen atom in the ring and which is unsubstituted or substituted by one or more substituents selected from OH, $C_{1-6}$alkyl, $C_{0-3}$alkylene-$C_{3-5}$cycloalkyl, O-$C_{1-6}$alkyl, $C_{1-6}$alkylene-O-$C_{1-6}$alkyl and $(CH_2)_{0-3}$-phenyl.

**4.** The compound of any of claims 1 to 3 wherein B represents N and D and E both represent CH.

**5.** The compound of any of claims 1 to 3 wherein D represents N and B and E both represent CH.

**6.** The compound of any of claims 1 to 5 wherein T is $NR^{12}R^{13}$.

**7.** The compound of claim 6 wherein $R^{12}$ and $R^{13}$ are independently from each other selected from H, $C_{1-3}$alkyl and $(CH_2)_{0-2}$-$C_{3-6}$cycloalkyl, wherein alkyl and cycloalkyl are unsubstituted or substituted by 1 to 3 $R^{4a}$.

**8.** The compound of any of claims 1 to 5 wherein T is selected from

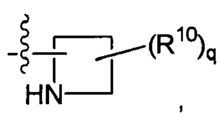 , 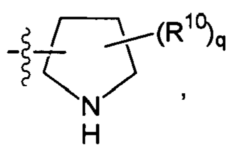 , 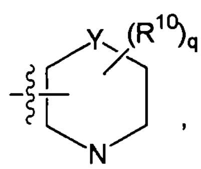 ,

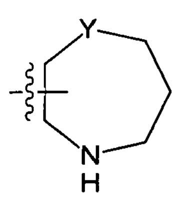

**9.** The compound of claim 8 wherein Y is $CH_2$ or $NR^{11}$ and $R^{10}$ is H, $NH_2$, $C_{1-6}$alkyl, $NH(C_{1-6}$alkyl) or $N(C_{1-6}$alkyl$)_2$.

**10.** The compound of any of claims 1 to 9, wherein
X is phenyl,

4 to 8-membered saturated or unsaturated heterocyclyl containing 1 to 4 heteroatoms independently selected

from N, O and S, or
5- to 6-membered heteroaryl containing 1 to 4 heteroatoms independently selected from N, O and S,

wherein each phenyl, heterocyclyl or heteroaryl is unsubstituted or substituted by 1 to 3 $R^{4a}$ and/or 1 $R^{4b}$ and/or 1 $R^5$.

**11.** The compound of any of claims 1 to 9, wherein
X is $-C(O)-R^6$,

$-OR^{14}$ or
$-NR^{15a}R^{15b}$.

**12.** The compound of claim 11, wherein $R^6$ is $-O-C_{1-6}$alkyl, wherein alkyl is unsubstituted or substituted with one or more $R^{16}$.

**13.** The compound of claim 11, wherein $R^6$ is $NR^{16a}R^{16b}$.

**14.** The compound of claim 13, wherein $R^6$ is $NH-C_{1-6}$alkyl, wherein alkyl is unsubstituted or substituted with one or more $R^{16}$.

**15.** The compound of any of claims 1 to 14 as a medicament.

**16.** The compound of any of claims 1 to 14 as a melanocortin-4 receptor antagonist.

**17.** The compound of any of claims 1 to 14 for the prophylaxis or treatment of disorders, diseases or conditions responsive to the inactivation of the melanocortin-4 receptor in a mammal.

**18.** The compound of claim 17 for the prophylaxis or treatment of cancer cachexia.

**19.** The compound of claim 17 for the prophylaxis or treatment of muscle wasting.

**20.** The compound of claim 17 for the prophylaxis or treatment of anorexia.

**21.** The compound of claim 17 for the prophylaxis or treatment of anxiety and/or depression.

**22.** The compound of claim 17 for the prophylaxis or treatment of emesis.

**23.** The compound of claim 17 for the prophylaxis or treatment of amytrophic lateral sclerosis (ALS).

**24.** Use of the compound of any of claims 1 to 14 for the preparation of a medicament for the prophylaxis or treatment of disorders, diseases or conditions responsive to the inactivation of the melanocortin-4 receptor in a mammal.

**25.** The use according to claim 24 for the preparation of a medicament for the prophylaxis or treatment of cancer cachexia.

**26.** The use according to claim 24 for the preparation of a medicament for the prophylaxis or treatment of muscle wasting.

**27.** The use according to claim 24 for the preparation of a medicament for the prophylaxis or treatment of anorexia.

**28.** The use according to claim 24 for the preparation of a medicament for the prophylaxis or treatment of anxiety and/or depression.

**29.** The use according to claim 24 for the preparation of a medicament for the prophylaxis or treatment of emesis.

**30.** The use according to claim 24 for the preparation of a medicament for the prophylaxis or treatment of amytrophic lateral sclerosis (ALS).

**31.** A pharmaceutical composition comprising a compound of any of claims 1 to 14 and a pharmaceutically acceptable carrier.

European Patent Office

# EUROPEAN SEARCH REPORT

Application Number
EP 08 00 5046

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2008/027812 A (FOREST LAB HOLDINGS LTD; ARALDI GIAN-LUCA [US]; RONSHEIM MATTHEW [US];) 6 March 2008 (2008-03-06) Diseases treatable listed on page 20, lines 4-21 and 21, line 4 * page 28 - page 29; examples 1,3 * ----- | 1-31 | INV. C07D487/04 A61P1/00 A61P35/00 A61P21/00 A61K31/519 A61K31/4985 A61K31/5025 |
| A | WO 03/070732 A (UPJOHN CO [US]; ROGERS BRUCE N [US]; PIOTROWSKI DAVID W [US]; WALKER D) 28 August 2003 (2003-08-28) * pages 114,118; claims 77,79; examples 18,29 * ----- | 1-31 | |
| | | | TECHNICAL FIELDS SEARCHED (IPC) |
| | | | C07D A61K A61P |

The present search report has been drawn up for all claims

| Place of search | Date of completion of the search | Examiner |
|---|---|---|
| The Hague | 12 August 2008 | Lange, Tim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

4

EPO FORM 1503 03.82 (P04C01)

## ANNEX TO THE EUROPEAN SEARCH REPORT ON EUROPEAN PATENT APPLICATION NO. EP 08 00 5046

This annex lists the patent family members relating to the patent documents cited in the above-mentioned European search report.
The members are as contained in the European Patent Office EDP file on
The European Patent Office is in no way liable for these particulars which are merely given for the purpose of information.

12-08-2008

| Patent document cited in search report | Publication date | Patent family member(s) | Publication date |
|---|---|---|---|
| WO 2008027812 A | 06-03-2008 | US 2008058350 A1 | 06-03-2008 |
| WO 03070732 A | 28-08-2003 | AU 2003219690 A1 | 09-09-2003 |
| | | BR 0307735 A | 25-01-2005 |
| | | CA 2476681 A1 | 28-08-2003 |
| | | EP 1476449 A1 | 17-11-2004 |
| | | JP 2005523288 T | 04-08-2005 |
| | | MX PA04008038 A | 26-11-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0074679 A
- WO 02066478 A


### Non-patent literature cited in the description


- **A. Kask et al.** Selective antagonist for the melanocortin-4 receptor (HS014) increases food intake in free-feeding rats. *Biochem. Biophys. Res. Commun.,* 1998, vol. 245, 90-93
- **Chen et al.** *Cell,* 1997, vol. 91, 789-798
- **S.Q. Giraudo et al.** Feeding effects of hypothalamic injection of melanocortin-4 receptor ligands. *Brain Research,* 1998, vol. 80, 302-306
- **D. Huszar et al.** *Cell,* 1997, vol. 88, 131-141
- **I. Corcos et al.** HP-228 is a potent agonist of melanocortin receptor-4 and significantly attenuates obesity and diabetes in Zucker fatty rats. *Society for Neuroscience Abstracts,* 1997, vol. 23, 673
- **H. Wessells et al.** Synthetic Melanotropic Peptide Initiates Erections in Men With Psychogenic Erectile Dysfunction: Double-Blind, Placebo Controlled Crossover Study. *J. Urol.,* 1998, vol. 160, 389-393
- **Owen MJ ; Nemeroff CB.** Physiology and pharmacology of corticotrophin releasing factor. *Pharmacol Rev,* 1991, vol. 43, 425-473
- **Shigeyuki Chaki et al.** *J. Pharm. Exp. Ther.,* 2003, vol. 304 (2), 818-26
- **Marks D.L. et al.** Role of the central melanocortin system in cachexia. *Cancer Res.,* 2001, vol. 61, 1432-1438
- **Ludolph AC.** *Neuromuscul. Disord.,* 2006, vol. 16 (8), 530-8
- **Chen, A.S. et al.** *Transgenic Res,* April 2000, vol. 9 (2), 145-54
- **Marks, D.L. ; Ling, N ; Cone, R.D.** *Cancer Res,* 15 February 2001, vol. 61 (4), 1432-8
- **Zhao, Y.H. et al.** Evaluation of Human Intestinal Absorption Data and Subsequent Derivation of a Quantitative Structure-Activity Relationship (QSAR) with the Abraham Descriptors. *Journal of Pharmaceutical Sciences,* 2001, vol. 90 (6), 749-784
- **Deferme, S. ; Mols, R. ; Van Driessche, W. ; Augustijns, P.** Apricot Extract Inhibits the P-gp-Mediated Efflux of Talinolol. *Journal of Pharmaceutical Sciences,* 2002, vol. 91 (12), 2539-48